Europäisches Patentamt

European Patent Office

Office européen des brevets

(19)

(11) **EP 1 067 969 B1**

(12) **FASCICULE DE BREVET EUROPEEN**

(45) Date de publication et mention
de la délivrance du brevet:
**23.07.2003 Bulletin 2003/30**

(51) Int Cl.⁷: **A61K 48/00**, C07H 17/08,
C12N 15/87, C12N 5/10,
A61P 31/00

(21) Numéro de dépôt: **99913358.0**

(22) Date de dépôt: **07.04.1999**

(86) Numéro de dépôt international:
**PCT/FR99/00808**

(87) Numéro de publication internationale:
**WO 99/051274 (14.10.1999 Gazette 1999/41)**

(54) **DERIVES DE MACROLIDES POLYENIQUES, UTILISATION POUR LA VECTORISATION DE MOLECULES**

POLYEN-MAKROLIDDERIVATE, VERWENDUNG ZUR VEKTORISIERUNG VON MOLEKÜLEN

POLYENE MACROLIDE DERIVATIVES, USE FOR VECTORING MOLECULES

(84) Etats contractants désignés:
**AT BE CH CY DE DK ES FI FR GB GR IE IT LI LU
MC NL PT SE**

(30) Priorité: **07.04.1998 FR 9804317**

(43) Date de publication de la demande:
**17.01.2001 Bulletin 2001/03**

(73) Titulaires:
• **UNIVERSITE PIERRE ET MARIE CURIE PARIS VI
75252 Paris Cédex 05 (FR)**
• **Technical University of Gdansk
80-952 Gdansk (PL)**

(72) Inventeurs:
• **BOLARD, Jacques
F-92150 Meudon (FR)**
• **GARCIA, Christine
F-75014 Paris (FR)**
• **SEKSEK, Olivier
F-75012 Paris (FR)**
• **BOROWSKI, Edward
PL-80-288 Gdansk (PL)**
• **GRZYBOWSKA, Jolanta
PL-80-269 Gdansk (PL)**

(74) Mandataire: **Desaix, Anne et al
Ernest Gutmann - Yves Plasseraud S.A.
3, rue Chauveau-Lagarde
75008 Paris (FR)**

(56) Documents cités:
**WO-A-93/17034**

• **DATABASE CHEMICAL ABSTRACTS [Online]
FILE SERVER STN KARLSRUHE abrégé
114:43402, CZERWINSKI: "Amphotericin B
2-morpholinoethylamide diaspartate, a new
water soluble derivative of the antibiotic
synthesis and biological properties"
XP002088733 & J.ANTIBIOT., vol. 43, no. 6, 1990,
pages 680-683,**
• **CHEMICAL ABSTRACTS, vol. 113, no. 23, 1990
Columbus, Ohio, US; abstract no. 208215h,
GRZYBOWSKA ET AL: "Hydrazides - a novel
type of derivatives of polyene macrolide
antifungal antibiotics" page 402; colonne 2;
XP002088732 & J. ANTIBIOT., vol. 43, no. 7, 1990,
pages 907-908,**
• **DATABASE MEDLINE [Online] FILE SERVER
STN KARLSRUHE ABR G 85168520, HIDAKA ET
AL: "Amphotericin B enhances efficiency of
DNA-mediated gene transfer in mammalian
cells" XP002088734 cité dans la demande &
SOMATIC CELL AND MOLECULAR GENETICS,
vol. 11, no. 2, 1985, pages 109-115,**
• **HARTSEL S ET AL: "Amphotericin B: new life for
an old drug." TRENDS IN PHARMACOLOGICAL
SCIENCES, (1996 DEC) 17 (12) 445-9. REF: 51
JOURNAL CODE: WFT. ISSN: 0165-6147.,
XP002088730 ENGLAND: United Kingdom cité
dans la demande**
• **WYMAN ET AL: "DESIGN, SYNTHESIS, AND
CHARACTERIZATION OF A CATIONIC PEPTIDE
THAT BINDS TO NUCLEIC ACIDS AND
PERMEABILIZES BILAYERS" BIOCHEMISTRY,
vol. 36, 1997, pages 3008-3017, XP002088731
cité dans la demande**

LISTE DE SEQUENCES

<110> UNIVERSITE PIERRE ET MARIE CURIE (PARIS VI) TECHNICAL UNIVERSITY OF GDANSK

<120> COMPOSITIONS POUR LA VECTORISATION DE MOLECULES

<130> B3850A - PB/KM

<140>
<141>

<150> 9804317
<151> 1998-07-04

<160> 2

<170> PatentIn Ver. 2.1

<210> 1
<211> 20
<212> ADN
<213> Séquence artificielle

<220>
<223> Description de la séquence artificielle: OLIGONUCLEOTIDE

<400> 1

ccatcccgac ctcgcgctcc                                                    20

<210> 2
<211> 27
<212> ADN
<213> Séquence artificielle

<220>
<223> Description de la séquence artificielle: OLIGONUCLEOTIDE

<400> 2

tgtgtttttg ttttgttggt tttgttt                                           27


## Description

La présente invention concerne le domaine de la biologie, et notamment du transfert de composés dans les cellules. Elle concerne plus particulièrement de nouveaux vecteurs et compositions pour le transfert de molécules d'intérêt, notamment d'acides nucléiques, dans les cellules in vitro, ex vivo ou in vivo. La présente invention possède de nombreuses applications dans les domaines de la biologie expérimentale, clinique ou médicale.

La possibilité de transférer efficacement des molécules d'intérêt dans les cellules constitue un des défis principaux dans le développement de la biologie et des biotechnologies. Ce transfert permet par exemple, in vitro, la réalisation d'expérimentations biologiques ou biochimiques (étude de régulation de l'expression de gènes, mutagenèse, création de plasmides; études génomiques, etc.), la production de protéines ou peptides recombinants, notamment d'intérêt pharmaceutique ou agro-alimentaire, ou encore la production de virus recombinants. Ex vivo ou in vivo, ce transfert peut permettre la réalisation d'études de marquage, de biodisponibilité, d'expression tissulaire, la création d'animaux transgéniques exprimant des gènes étrangers par exemple, ou encore des applications biologiques ou médicales (vaccinations, thérapies, etc.).

Il est donc particulièrement important de pouvoir disposer d'un système de transfert de molécules d'intérêt efficace, applicable aux populations de cellules visées, et non-toxique pour les cellules ou les organismes utilisés.

Différentes approches ont été développées dans l'art antérieur pour transférer des molécules d'intérêt dans les cellules. Il s'agit par exemple de vecteurs d'origine virale, dont l'efficacité de transfert d'acides nucléiques dans les cellules est remarquable. Néanmoins, ce type de vecteur présente également certains inconvénients potentiels, liés notamment à leur préparation, leur innocuité, leur capacité de clonage, etc.

Parallèlement à ces vecteurs viraux, de très nombreux systèmes synthétiques ont été avancés pour la vectorisation (i.e., le transfert) de molécules d'intérêt, notamment des acides nucléiques. Ces systèmes synthétiques sont, la plupart du temps, cationiques de manière à interagir avec les acides nucléiques, chargés négativement (Legendre, 1996). Toutefois, dans la plupart des cas (polylysine, polymères cationiques, polyamines, etc.), il est considéré que les complexes acides nucléiques/vecteurs pénètrent par endocytose, ce qui pose le problème (i) du relargage ultérieur des acides nucléiques hors des vésicules d'endocytose afin de leur permettre d'atteindre la cible et (ii) de la dégradation des acides nucléiques dans ces compartiments cellulaires. En outre, ces systèmes ne procurent pas toujours une efficacité satisfaisante in vitro.

Pour remédier à ce problème, il a été proposé d'ajouter différents adjuvants aux formulations initiales, de manière à court-circuiter l'endocytose et permettre une libération directe de l'acide nucléique dans le cytoplasme. Les auteurs envisagent en général un mécanisme d'entrée par fusion de ces complexes avec les membranes endosomales ou plasmiques (ou par déstabilisation transitoire de ces membranes). Sont très utilisés, par exemple des peptides fusiogènes ou la dioléoylphosphatidylcholine (DOPE). Récemment, des effecteurs induisant spécifiquement une perméabilité membranaire ont été envisagés. Il s'agit d'abord de la gramicidine S (ou de la tyrocidine) associée à des vésicules de DOPE (Legendre et Szoka, 1993) formulation néanmoins inactive en présence de sérum. On trouve aussi un peptide perturbant la perméabilité membranaire à bas pH, associé à un autre peptide amphiphile (Ohmori, BBRC, 1997). Cette formulation est moins active que la Lipofectine®, composé de référence, et présente une toxicité non négligeable.

Ces dernières formulations présentent en outre l'inconvénient d'avoir plusieurs composants, ce qui donne des mélanges hétérogènes et instables dans les fluides biologiques.

Pour ce qui est des systèmes à un seul composant on trouve, toujours dans le but d'agir sur la perméabilité membranaire, un peptide amphipatique cationique (Wyman, 1997) susceptible de causer la fuite transmembranaire au bas pH rencontré dans les endosomes. On trouve aussi la dioléoylmélittine (Legendre 1997) qui présente l'avantage d'être active en présence de sérum.

En dépit des efforts entrepris, les systèmes décrits dans l'art antérieur présentent des inconvénients liés à une faible activité in vivo, des effets secondaires in vivo, des spectres d'activité étroits in vitro, et/ou des coûts prohibitifs.

La présente invention concerne un nouveau système de vectorisation de molécules d'intérêt dans les cellules. Le système de l'invention est adapté à la vectorisation (au transfert) dans les cellules de molécules chargées négativement, plus particulièrement d'acides nucléiques.

Le système de l'invention repose plus particulièrement sur la mise au point et/ou l'utilisation de composés comprenant une partie cationique, susceptible d'interagir avec les molécules d'intérêt chargées négativement, et une partie active particulière permettant le transfert dans les cellules. Plus particulièrement, les composés utilisés dans la présente invention sont des composés macrolides polyéniques cationiques, comprenant des antibiotiques macrolides polyéniques et des dérivés (en particulier cationiques) de ceux-ci.

Les compositions selon l'invention présentent l'avantage d'une préparation simple, d'une faible toxicité, d'une activité conservée en présence de sérum, d'une bonne efficacité, et d'une compatibilité avec un usage pharmacologique.

La présente invention décrit également de nouveaux composés dérivés d'antibiotiques macrolides polyéniques (tels que de l'amphotéricine B), utilisables notamment pour la vectorisation de molécules d'intérêt ou comme agent(s) antifongique(s).

La présente invention apporte donc une alternative aux systèmes décrits dans l'art antérieur pour la vectorisation de molécules d'intérêt.

Un premier objet de l'invention réside donc dans une composition comprenant :

- une molécule d'intérêt chargée négativement, et,
- un composé macrolide polyénique cationique susceptible d'interagir avec ladite molécule.

Comme indiqué plus haut, l'invention vise des composés ou compositions permettant le transfert dans les cellules de "molécules d'intérêt". Il s'agit essentiellement de molécules chargées négativement; compte tenu du caractère cationique des composés de vectorisation utilisés. On peut citer à titre d'exemple de telles molécules chargées négativement des protéines, peptides ou polypeptides, les PNA ou les acides nucléiques par exemple.

Il s'agit avantageusement d'acides nucléiques. A cet égard, les acides nucléiques peuvent être des acides désoxyribonucléiques (ADN) ou ribonucléiques (ARN). S'agissant d'ADN, on peut citer des ADN complémentaire, génomique, synthétiques ou semi-synthétiques. Les ARN peuvent être des ARN messagers, de transfert, ribosomaux ou synthétiques. Les acides nucléiques peuvent être d'origine humaine, animale, végétale, virale, bactérienne ou artificielle par exemple. Il peut s'agir d'oligonucléotides, (ayant par exemple une longueur comprise entre 3 et 80-mères), ou de phases codantes, gènes, régions génomiques ou chromosomes entiers. Il peut s'agir d'acides nucléiques simple- ou double-brin. Il peut s'agir en particulier d'acides nucléiques antisens (ou anti-gène), c'est-à-dire capables d'interférer, par hybridation, avec l'activité d'un gène ou d'un ARN ou d'une région génomique particulière. Il peut également s'agir d'acides nucléiques comprenant une région codant pour un produit peptidique d'intérêt (polypeptide ou protéine ayant une activité biologique intéressante sur le plan immunologique, pharmaceutique ou alimentaire). Par ailleurs, ces acides nucléiques peuvent être sous forme de plasmide, linéaire ou circulaire.

Les acides nucléiques peuvent être obtenus par toutes les techniques connues de l'homme du métier, telles que la synthèse artificielle, le criblage de banques, l'isolement à partir de plasmides, etc., ainsi que toute combinaison de ces techniques classiques ou de toute autre technique de la biologie moléculaire.

Le molécule chargée négativement peut également être une protéine, un polypeptide ou un peptide, notamment un peptide antigénique.

Comme indiqué ci-avant, le système de l'invention repose avantageusement sur l'utilisation d'antibiotiques macrolides polyéniques et de dérivés (en particulier cationiques) de ceux-ci.

La famille des antibiotiques macrolides polyéniques comporte essentiellement deux groupes : les composés macrolides polyéniques aromatiques et les composés macrolides polyéniques non aromatiques. Les premiers sont souvent isolés de microorganismes sous une forme portant une certaine charge nette positive à pH neutre. Parmi ceux-ci, on peut citer notamment les composés macrolides heptaéniques suivants : hamycine, trichomycine, candicidine, vacidine A (syn. partricine B), gédamycine (syn. partricine A) ou encore la périmycine, notamment la périmycine A. Les composés du second groupe (non-aromatiques) sont des composés zwitterioniques, et donc globalement neutres, à l'exception de la liénomycine. Dans ce second groupe, entre notamment l'amphotéricine B. L'amphotéricine B est couramment utilisée en clinique comme antifongique sous forme de Fungizone® (amphotéricine B associée à du désoxycholate). L'amphotéricine B est par ailleurs connue pour interagir avec les membranes contenant des stérols et pour former, à concentration sublétale, des pores transmembranaires aussi bien dans les cellules fongiques que, à concentration plus forte, dans les cellules de mammifères (Hartsel et Bolard, 1996).

Néanmoins, l'utilisation de ces composés macrolides polyéniques ou de dérivés cationiques de ceux-ci, en particulier de composés macrolides heptaéniques cationiques, pour la vectorisation de molécules d'intérêt n'a jamais été rapportée.

En particulier, nombre des antibiotiques macrolides polyéniques sont globalement non chargés et, de ce fait, ne peuvent pas permettre la réalisation d'un complexe acide nucléique/vecteur. Tel est le cas par exemple de l'amphotéricine B, qui, en tant que telle, ne semble pas capable d'interagir avec les acides nucléiques. La présente invention montre maintenant qu'il est possible d'utiliser des composés macrolides polyéniques, naturels, synthétiques ou hémisynthétiques, porteurs d'un groupement chargé positivement pour le transfert de molécules négatives, notamment d'acides nucléiques. La présente invention montre en particulier qu'il est possible d'utiliser des dérivés de composés macrolides polyéniques, tels l'amphotéricine B par exemple, pour former des complexes avec des acides nucléiques, et que ces complexes peuvent être vectorisés efficacement dans les cellules, c'est-à-dire que (i) une liaison électrostatique s'établit entre ce composé macrolide et les charges négatives de l'acide nucléique, et (ii) la partie polyénique du complexe conserve son activité membranaire.

La présente invention montre également que de tels complexes permettent de protéger les acides nucléiques de la dégradation par le sérum, et donc d'augmenter l'efficacité du transfert dans des conditions de sérum, notamment in vivo ou ex vivo.

Selon un mode particulier de réalisation, l'invention concerne une composition telle que définie ci-avant dans laquelle le composé macrolide polyénique cationique est un antibiotique macrolide heptaénique aromatique. Avantageusement, le composé est la périmycine.

Selon un autre mode particulier de réalisation, l'invention concerne une composition telle que définie ci-avant dans laquelle le composé macrolide polyénique cationique est un dérivé d'antibiotique macrolide polyénique comprenant un ou plusieurs groupes fonctionnels cationiques.

Le terme "dérivé" désigne au sens de l'invention toute forme modifiée chimiquement par introduction au moins d'un groupe fonctionnel cationique.

Par ailleurs, l'expression "groupe fonctionnel cationique" inclut tout groupe susceptible de devenir cationique par protonation, selon le milieu utilisé (notamment le pH du milieu) ainsi que tout groupe porteur d'une ou plusieurs charges positives permanentes. Comme indiqué ci-avant, le ou les composés utilisés dans le cadre de la présente invention sont susceptibles d'interagir avec la molécule d'intérêt du fait de la présence de groupes fonctionnels cationiques. Ces groupes sont généralement liés de manière covalente sur le composé macrolide polyénique. La fonction cationique des composés utilisés dans l'invention peut être composée plus particulièrement de tout groupement chargé positivement susceptible d'interagir avec la ou les molécules d'intérêt. L'expression "susceptible d'interagir" signifie que la partie cationique peut s'associer avec la molécule d'intérêt anionique, notamment un acide nucléique. L'interaction entre les composés et l'acide nucléique est essentiellement une interaction ionique, non-covalente, de type liaison électrostatique, qui s'établit entre les charges positives de la partie cationique du composé et les charges négatives de la molécule d'intérêt (l'acide nucléique). A cette interaction ionique peuvent s'ajouter des interactions de type van der Waals ou hydrophobe. Le caractère non-covalent de l'interaction est avantageux dans la mesure où il permet la dissociation du complexe dans la cellule, et donc la libération des molécules d'intérêt dans les cellules. En outre, ce type de liaison permet une mise en oeuvre simplifiée puisqu'il est suffisant de mettre en présence les composés et les molécules d'intérêt pour former les complexes.

A la différence des systèmes dans lesquels l'acide nucléique est directement lié à la partie active de l'effecteur membranaire et doivent donc inhiber sa spécificité, la présente invention décrit une formulation où l'acide nucléique est lié au vecteur, également d'une manière électrostatique, mais laisse libre la partie active ce qui peut permettre de garder intacte son efficacité. De plus, aux concentrations utilisées, l'activité perméabilisante obtenue avec les compositions de l'invention est sans doute transitoire et ne doit pas être toxique pour la cellule.

Plus préférentiellement, dans les compositions de l'invention, le composé est un dérivé d'antibiotique macrolide heptaénique. Il s'agit plus particulièrement d'un dérivé cationique de l'amphotéricine B, de la candidine, de la mycoheptine ou de la vacidine. Il peut s'agir aussi d'un dérivé de la nystatine. Plus préférentiellement encore, le composé est un dérivé d'antibiotique macrolide heptanéique non aromatique, de préférence un dérivé de l'amphotéricine B.

Préférentiellement, le composé utilisé porte au moins deux charges positives. Les groupements fonctionnels chargés positivement qui peuvent être liés sur les composés macrolides polyéniques sont par exemple des esters, (poly)amides, hydrazide, polyamines, N-alkyl, N-amino acyl, polylysines, guanidines, ou des combinaisons de ceux-ci, ou plus généralement tout groupement hydrocarboné possédant une ou plusieurs charges positives, qui peuvent être apportées notamment par des atomes d'azote. On peut citer à titre d'exemples spécifiques de partie cationique adaptée pour les composés de l'invention les groupes alkylester (par exemple méthyl-, éthyl- ou propylester), alkylammonium (notamment triméthylammonium), lysil, ornithyl, guanidino, amidique, spermidique.

Le ou les groupes fonctionnels cationiques peuvent être liés sur le composé macrolide en différentes positions. Dans un premier mode de mise en oeuvre, la partie cationique est introduite sur une fonction amine du macrolide polyénique, et en particulier sur la fonction aminée du groupe mycosamine de la molécule. Dans un autre mode de mise en oeuvre, la partie cationique est introduite sur la fonction acide carboxylique du groupe aglycone de la molécule. Il est par ailleurs possible d'introduire la partie cationique par modification de ces deux positions de la molécule (voir Schnaffner et al. (1987) incorporé à la présente par référence, ainsi que les exemples qui suivent). Il est également entendu que la partie cationique peut être insérée sur d'autres positions de la molécule.

Dans un mode particulier de mise en oeuvre, l'invention concerne donc une composition telle que définie ci-dessus dans laquelle le dérivé d'antibiotique macrolide polyénique comprend un ou plusieurs groupes fonctionnels cationiques liés de manière covalente sur la fonction carboxylique du groupe aglycone et/ou sur la ou les fonctions amine de l'antibiotique macrolide polyénique.

Par ailleurs, il est entendu que le composé dérivé d'antibiotique macrolide polyénique selon l'invention peut comprendre, en plus de groupe(s) fonctionnel(s) cationique(s), d'autre(s) modification(s) structurale(s), dès lors que le composé obtenu conserve son activité, c'est-à-dire (i) la capacité d'interagir avec les acides nucléiques et (ii), la capacité de transférer des molécules. Cette dernière propriété permet en effet aux composés de l'invention de délivrer des molécules d'intérêt aux cellules, éventuellement par perméabilisation membranaire sans passer par la voie endocytique. Cette propriété est assurée dans les composés de l'invention par l'utilisation originale de macrolides polyéniques, tels que l'amphotéricine B ou ses dérivés. L'utilisation de cette partie active originale pourrait permettre avantageusement d'induire la perméabilisation transitoire des cellules pour permettre le passage des molécules d'intérêt.

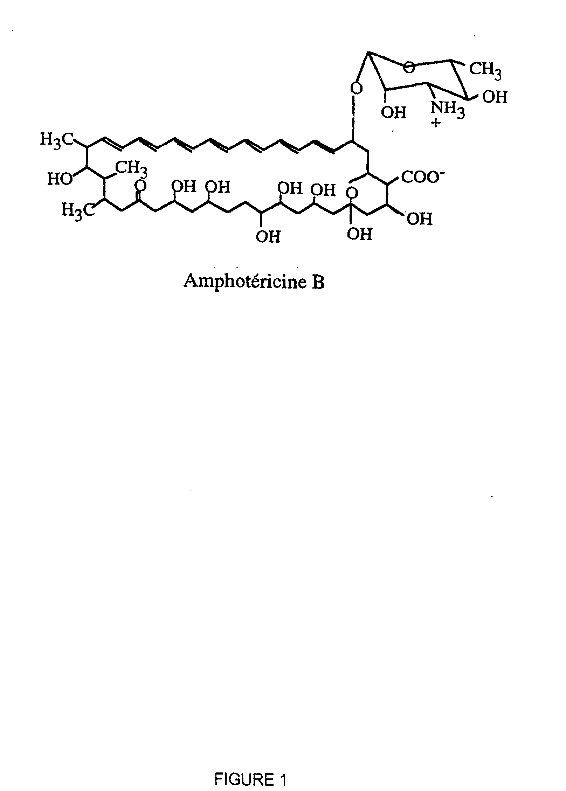

L'utilisation des composés selon l'invention est donc particulièrement avantageuse puisque les cellules ne sont pas perturbées de manière significative par le transfert de la molécule. Cette "partie active" peut en particulier être constituée de l'amphotéricine B (dont la formule est représentée sur la Figure 1), ou de tout variant de celle-ci ou antibiotique heptaénique macrolide, possédant des propriétés de solubilité, de toxicité, de biodisponibilité et/ou de perméabilisation modifiées. De tels variants ont été décrits par exemple dans Schaffner et al (1987), Malewicz et al. (1980) ; Binet et al. (1988), Chéron et al., (1989) ou encore Brajtburg et al. (1990) notamment, qui sont incorporés à la présente par référence.

Dans un mode particulièrement préféré de mise en oeuvre, la présente invention concerne une composition comprenant une molécule d'intérêt chargée négativement, telle qu'un acide nucléique, et un dérivé cationique de l'amphotéricine B.

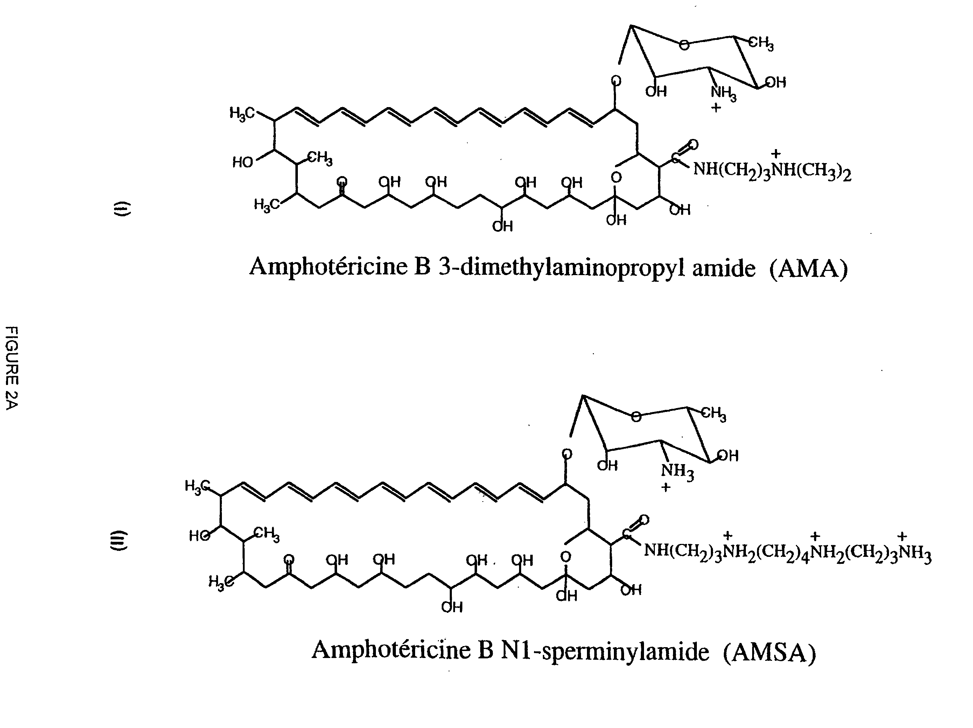

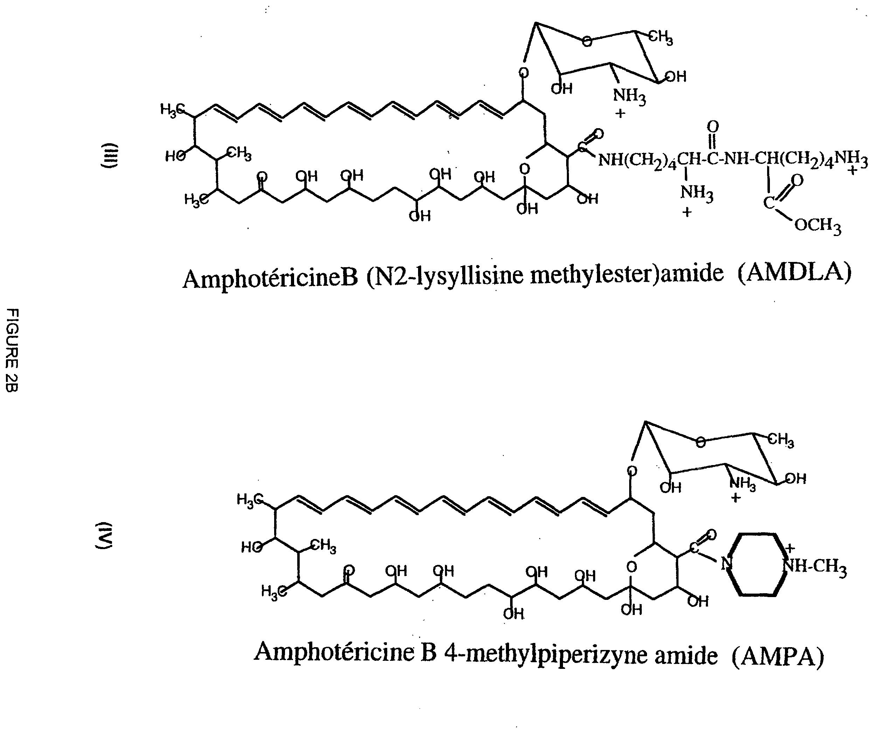

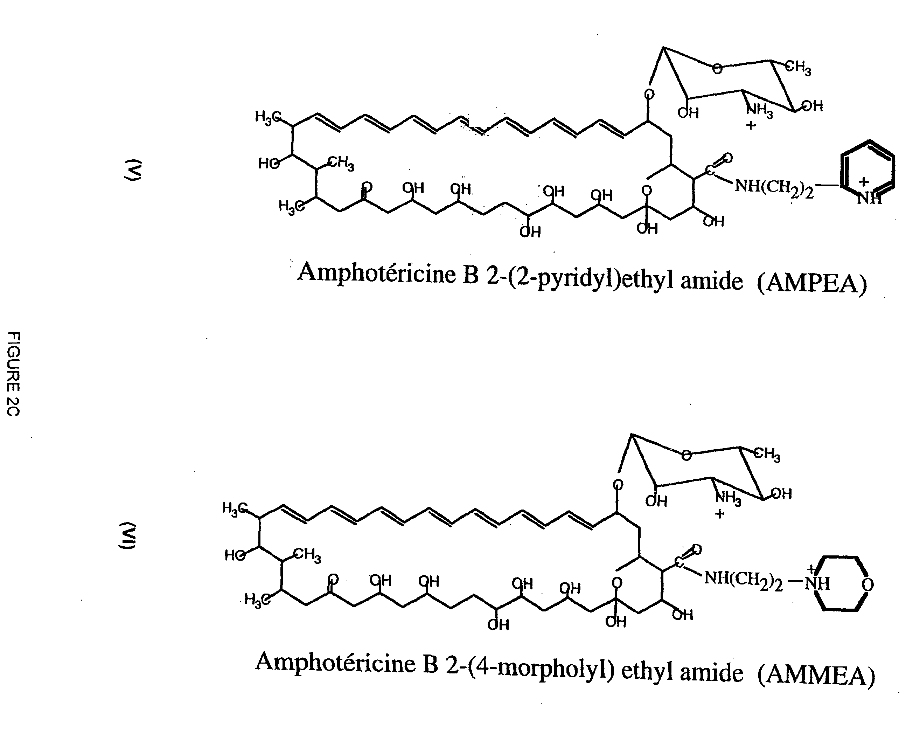

Selon une première variante particulière, le dérivé cationique est un amide primaire, secondaire ou tertiaire de l'amphotéricine B (voir Figure 2). A cet égard, on peut citer plus particulièrement les aminoalkyl amides, tels le diméthylaminopropyl amide, les amides de polyamines en particulier de la spermine, les amides de polyaminoacyl esters, notamment de dilysil methylester, ou encore les amides d'amines hétérocycliques telles que de N-méthyl pipérazine, 2-pyridyl éthyl amine ou 2-morpholine éthylamine.

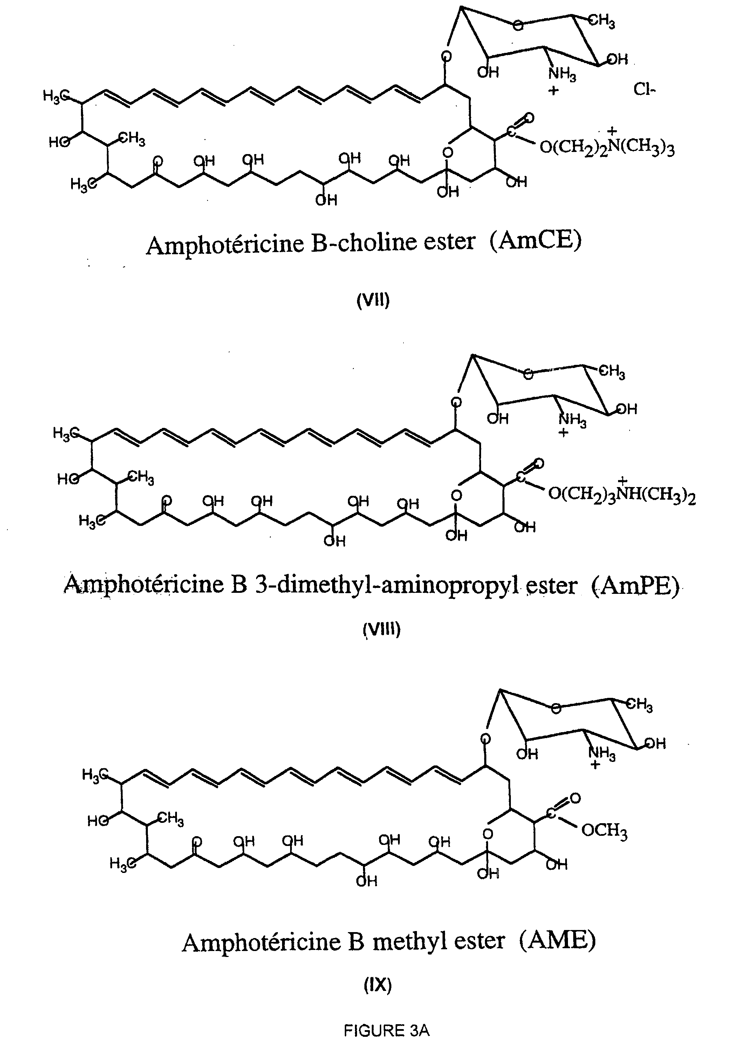

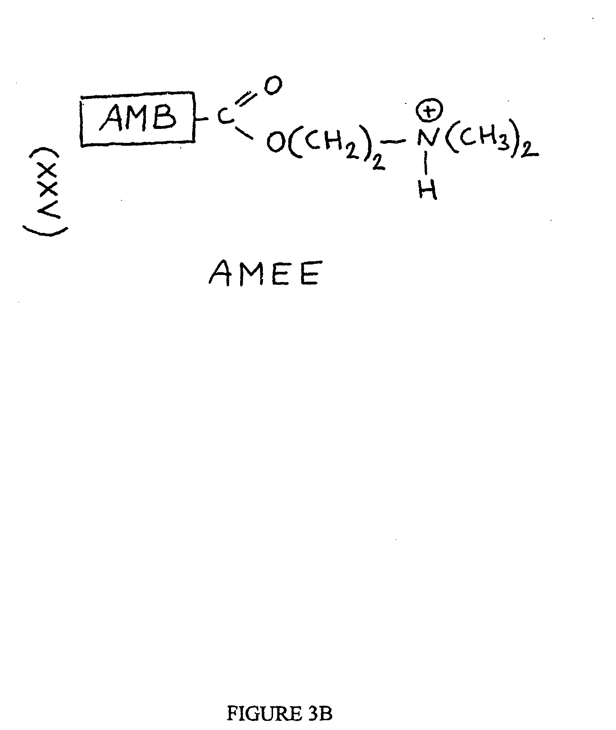

Selon une autre variante particulière, le dérivé cationique est un ester de l'amphotéricine B, de préférence un choline ester ou un diméthylaminopropyl ester (voir Figure 3).

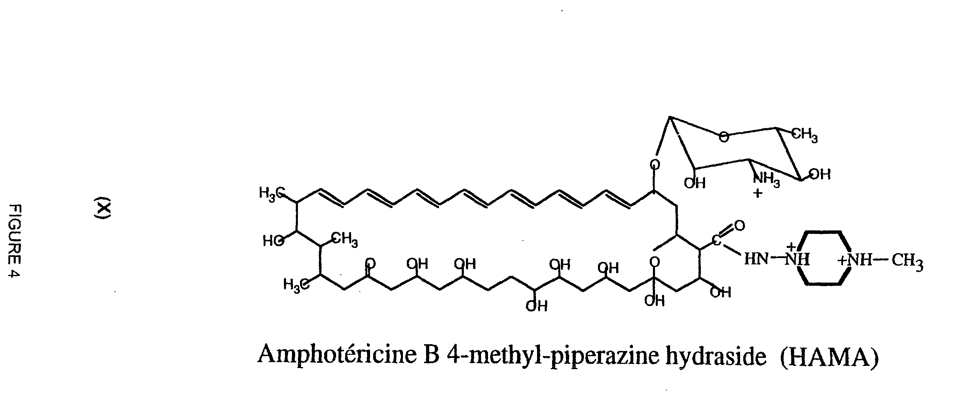

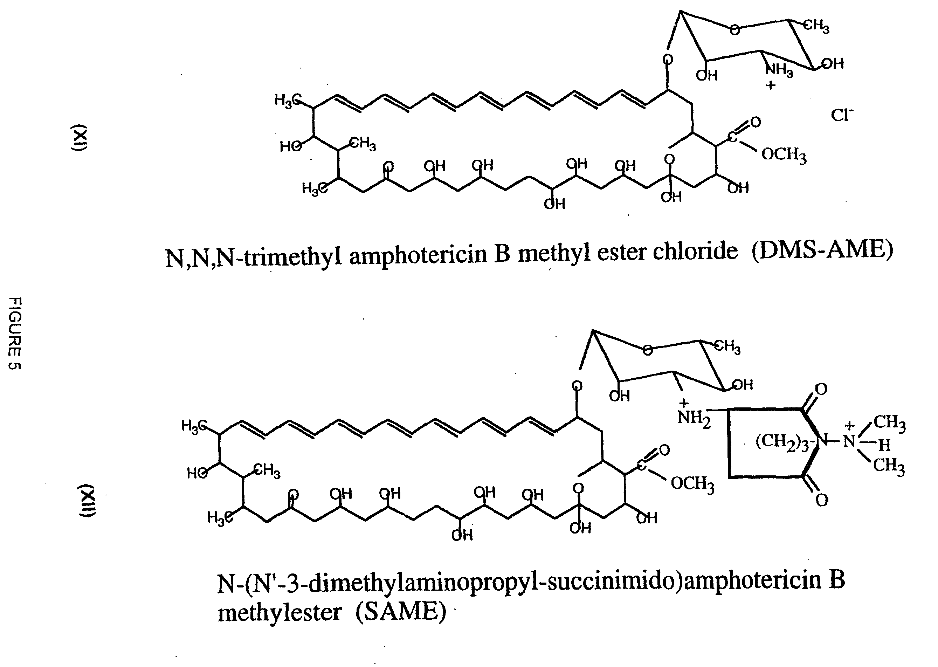

Selon une variante supplémentaire particulière, le dérivé cationique est un hydrazide de l'amphotéricine B, de préférence le N-méthyl pipérazine hydrazide de l'amphotéricine B (voir Figure 4).

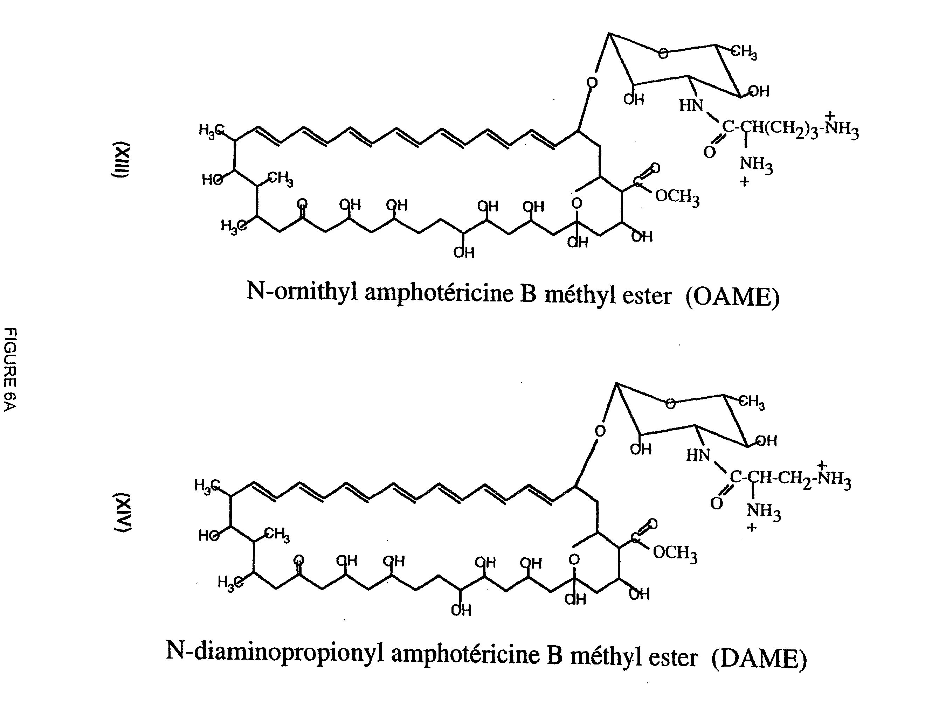

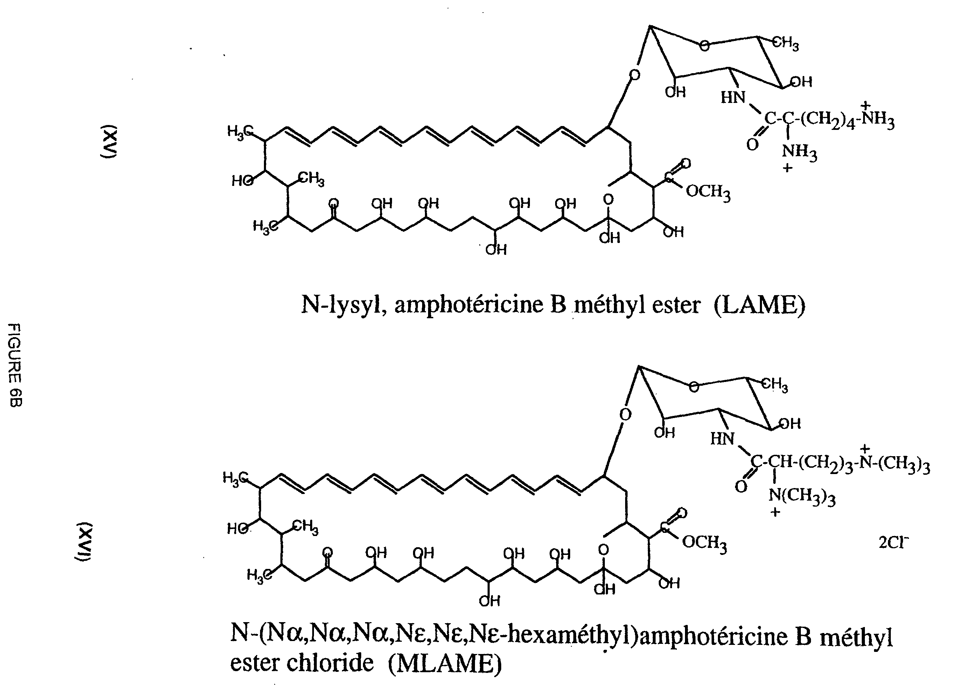

Toujours selon une variante particulière, le dérivé cationique est un dérivé N-alkyl de l'amphotéricine B, de préférence un dérivé N',N',N'-triméthyl ou N',N'-diméthylaminopropyl succinimidyl de l'amphotéricine B méthyl ester (voir Figure 5).

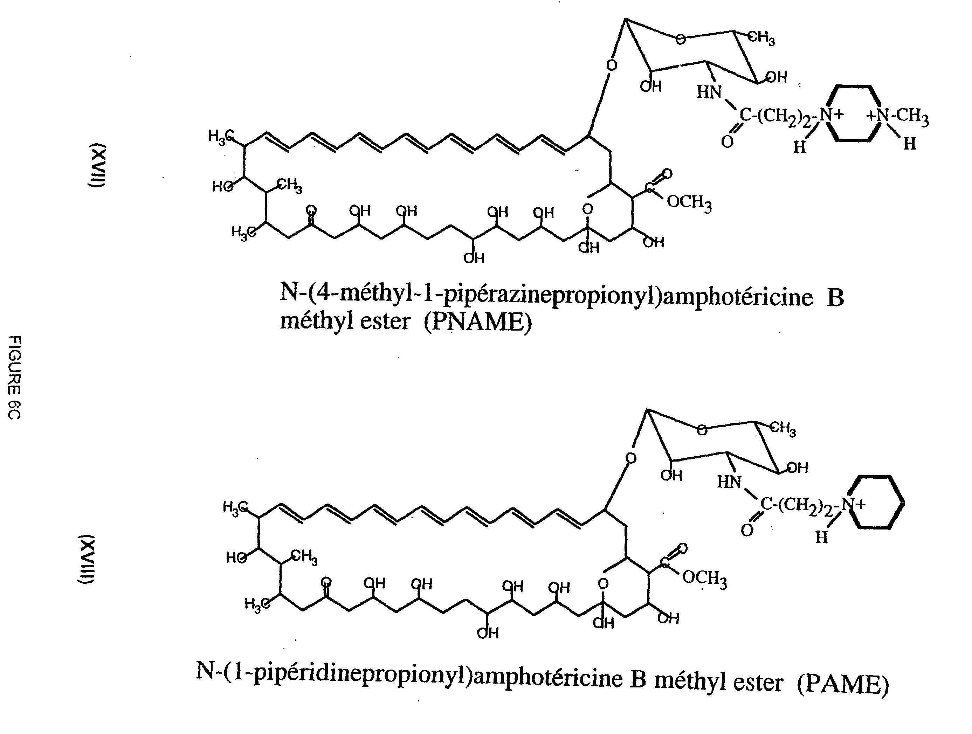

Dans une autre variante particulière, le dérivé cationique est un dérivé N-aminoacyl de l'amphotéricine B, de préférence le N-ornithyl-, N-diaminopropionyl-, N-lysil-, N-hexaméthyllysil-, N-tétraméthyllysil-, N-pipéridine-propionyl-, N-pipéridine-acétyl-, N'N'-méthyl-1-pipérazine-propionyl- et N,N'-méthyl-1-pipérazine-acétyl-amphotéricine B méthylester (voir Figure 6).

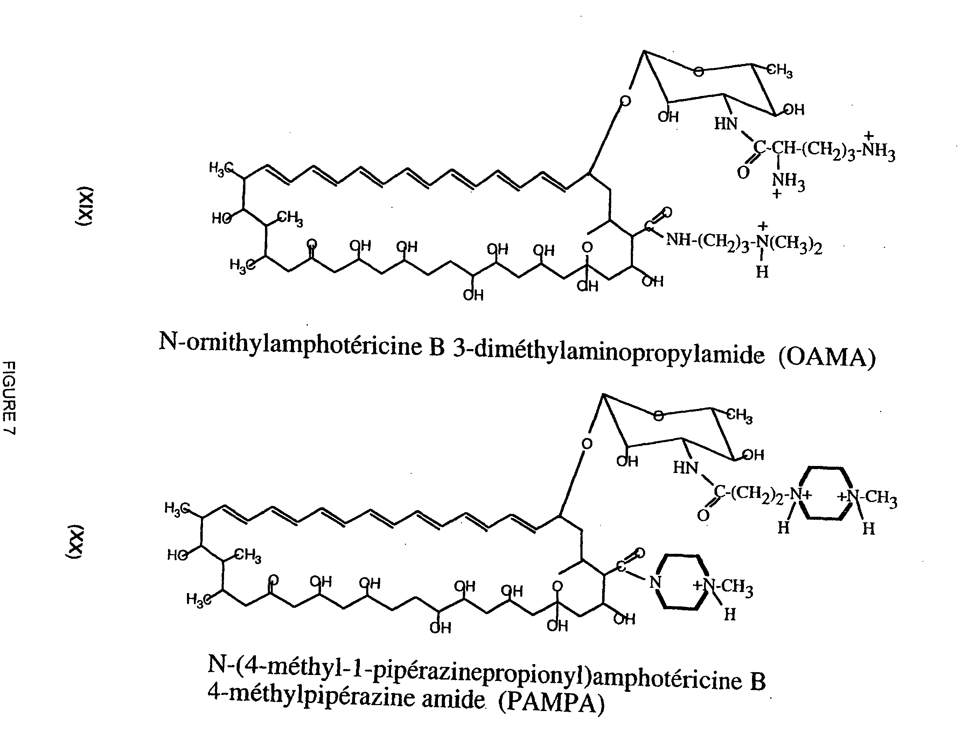

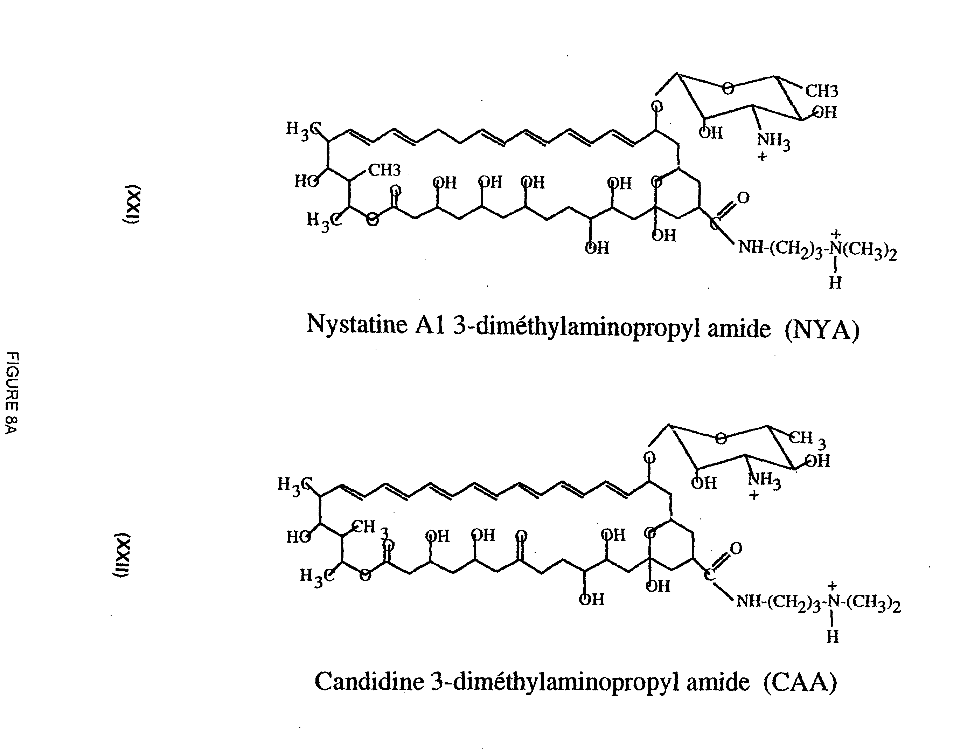

Dans un mode de réalisation particulier de l'invention, le dérivé cationique de l'amphotéricine B comporte un groupe cationique fonctionnel simultanément sur les positions carboxyl et amino de l'amphotéricine B (voir Figure 7). De tels dérivés sont par exemple le N-ornithyl amphotéricine B diméthylamino propyl amide ou le N-piperidino acétyl amphotéricine B-(-4-méthyl)piperazide.

Par ailleurs, dans les compositions de l'invention, les composés ci-dessus peuvent être sous forme de sels, tels que notamment de chlorure, aspartate, glutamate ou ascorbate.

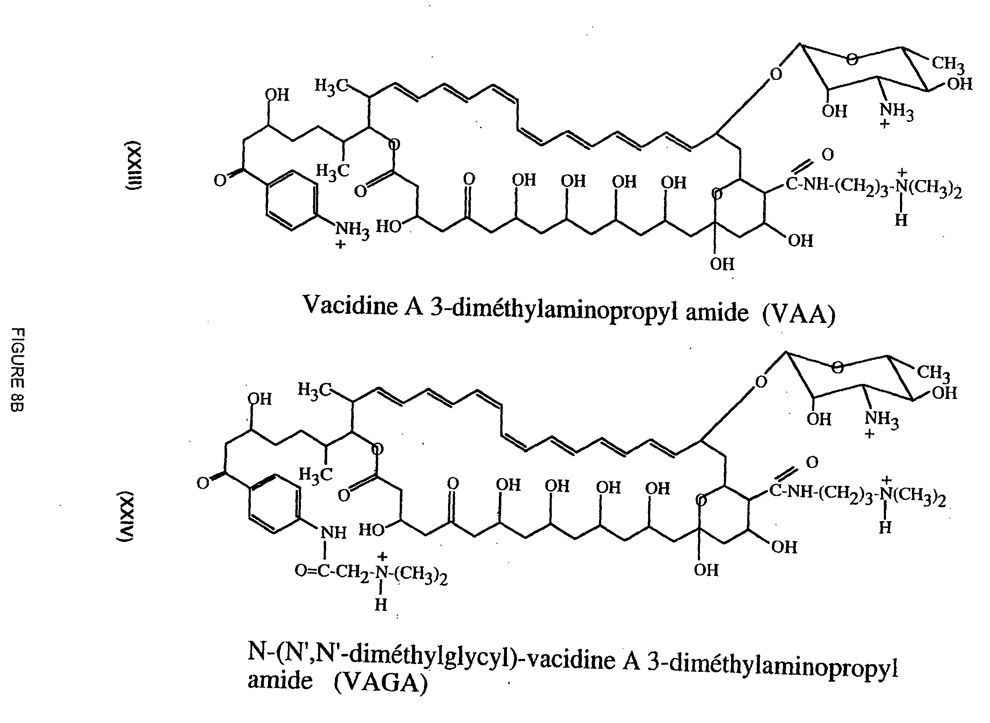

Dans un autre mode préféré de mise en oeuvre, la présente invention concerne une composition comprenant une molécule d'intérêt chargée négativement, telle qu'un acide nucléique, et un dérivé cationique d'un antibiotique macrolide heptaénique choisi parmi la nystatine, la candidine et la vacidine (voir Figure 8). De tels composés sont par exemple l'amide de la diméthylaminopropyl nystatine, l'amide de la diméthylaminopropyl candidine, l'amide de la diméthylaminopropyl vacidine ou le N'-diméthylaminoacétyl-vacidine-diméthylamino propylamide.

Des exemples particuliers de composés préférés selon l'invention sont :

- l'amphotéricine B diméthylaminopropylamide (AMA). L'AMA correspond au composé (I) représenté sur la Figure 2. Ce composé est constitué de l'amphothéricine B sur laquelle un groupe cationique diaminé (HN-(CH2)3-NH(CH3)2 a été lié en position 16 (fonction acide carboxylique du groupe aglycone). Ce composé comporte deux charges cationiques.

- l'amphotéricine B N1-sperminylamide (AMSA). L'AMSA correspond au composé (II) représenté sur la Figure 2.

- l'amphotéricine B (N$^2$-lysyllysine methylester)amide (AMDLA). L'AMDLA correspond au composé (III) représenté sur la Figure 2.

- l'amphotéricine B-4-méthylpiperizyne amide (AMPA). L'AMPA correspond au composé (IV) représenté sur la Figure 2.

- l'amphotéricine B 2-(2-piridyl)ethyl amide (AMPEA). L'AMPEA correspond au composé (V) représenté sur la Figure 2.

- l'amphotéricine B-2-(4-morpholyl) ethyl amide (AMMEA). L'AMMEA correspond au composé (VI) représenté sur la Figure 2.

- l'amphotéricine B choline ester (AMCE). L'AMCE correspond au composé (VII) représenté à la Figure 3.

- l'amphotéricine B 3-diméthyl-aminopropyl ester (AMPE). L'AMPE correspond au composé (VIII) représenté sur la Figure 3.

- l'amphotéricine B méthylester (AME). L'AME correspond au composé (IX) représenté à la Figure 3. Ce composé est constitué de l'amphotéricine B dans laquelle la fonction acide carboxylique du groupe aglycone a été estérifiée. Ce composé comporte une charge cationique.

- L'ester 2-diméthylaminoéthyl de l'amphotéricine B (AMEE). L'AMEE correspond au composé (XXV) représenté sur la Figure 3.

- l'amphotéricine B-4-méthyl-pipérazine hydrazide (HAMA). L'HAMA. correspond au composé (X) représenté à la Figure 4.

- N,N,N-triméthylammonium AME (DMS-AME). Le DMS-AME correspond au composé (XI) représenté sur la Figure 5. Ce composé est constitué de l'amphotéricine B sur laquelle deux groupes cationiques ont été greffés : un groupe méthyester en position 16 (fonction acide carboxylique du groupe aglycone) et un groupe triméthylammonium en position 19 (sur le fonction amine du groupe mycosamine). Ce composé comporte deux charges cationiques.

- N-(N'-3-diméthylaminopropyl-succinimido) amphotéricine B méthyl ester (SAME). Le SAME correspond au composé (XII) représenté sur la Figure 5.

- N-ornithyl AME (OAME). L'OAME correspond au composé (XIII) représenté sur la Figure 6.

- N-diaminopropionyl AME. Le DAME correspond au composé (XIV) représenté sur la Figure 6.

- N-lysil-AME (LAME). Le LAME correspond au composé (XV) représenté sur la Figure 6. Ce composé est constitué de l'amphotéricine B sur laquelle deux groupes cationiques ont été greffés : un groupe méthyester en position 16 (fonction acide carboxylique du groupe aglycone) et un groupe diaminé en position 19 (sur le fonction amine du groupe mycosamine). Ce composé comporte deux charges cationiques.

- N-(Nα,Nα,Nα,Nε,Nε,Nε-hexaméthyl) AME (MLAME). Le MLAME correspond au composé (XVI) représenté sur la Figure 6.

- N-(4-méthyl-1-piperazinepropionyl) AME (PNAME). Le PNAME correspond au composé (XVII) représenté sur la Figure 6.

- N-(1-pipéridinepropionyl) AME (PAME). Le PAME correspond au composé (XVIII) représenté sur la Figure 6.

- N-ornithyl-AMA (OAMA). L'OAMA correspond au composé (XIX) représenté sur la Figure 7.

- N-(4-méthyl-1-piperazinepropionyl) AMPA (PAMPA). Le PAMPA correspond au composé (XX) représenté sur la Figure 7.

- Nystatine A1 3-diméthylaminopropyl amide (NYA). Le NYA correspond au composé (XXI) représenté sur la Figure 8.

- Candidine 3-diméthylaminopropyl amide (CAA). Le CAA correspond au composé (XXII) représenté sur la Figure 8.

- Vacidine A 3-diméthylaminopropyl amide (VAA). Le VAA correspond au composé (XXIII) représenté sur la Figure 8.

- N-(N',N'-diméthylglycyl)-vacidine A 3-diméthylaminopropyl amide (VAGA). Le VAGA correspond au composé (XXIV) représenté sur la Figure 8.

Comme illustré dans les exemples, ces composés chargés positivement et notamment ceux possédant au moins 2 charges positives tels l'AMA, ont permis d'obtenir les résultats suivants :

- par interaction avec le composé, les oligonucléotides sont protégés de la dégradation induite par le sérum ;
- l'internalisation cellulaire d'oligonucléotides marqués à la fluorescéine dans les cellules (MCF7 et 3T3), est fortement augmentée par le composé. En particulier, comparée à l'action d'un composé de référence (la Lipofectine®), l'AmA induit une distribution intracellulaire homogène et non pas ponctuée, et la majorité de la population cellulaire est ciblée ;
- l'expression du gène MDR1 dans les fibroblastes transfectés par MDR1, est fortement réduite par des oligonucléotides phosphorothioates antisens anti-MDR1 vectorisés par le composé ;
- le composé permet aussi la vectorisation de gènes : nous avons pu, grâce à son utilisation, transfecter le gène GFP (green fluorescent protein).

Ces exemples montrent que les composés de l'invention tels que l'AmA, présentent des caractéristiques intéressantes de vecteur pour le transfert d'acides nucléiques dans les cellules (gènes, oligodesoxyribonucléotides antisens ou anti-gène).

Dans les compositions de l'invention, les quantités respectives de composé et de molécule(s) d'intérêt (par exemple d'acide nucléique) sont de préférence choisies de sorte que le rapport (R) des charges positives du composé sur les charges négatives de la molécule soit compris entre 0,1 et 20. Plus préférentiellement, ce rapport est compris entre 0,5 et 15. Il est entendu que ce rapport peut être adapté par l'homme du métier en fonction de la molécule d'intérêt (en particulier de l'acide nucléique) et du composé utilisés, des applications envisagées et du type cellulaire visé.

Les compositions de l'invention sont généralement préparées par incubation (par exemple mise en contact, en solution) du/des composés avec le/les molécules d'intérêt (acides nucléiques) pendant une période de temps suffisante pour permettre leur interaction. Le temps d'incubation est également fonction des composés utilisés et des conditions de l'incubation (milieu, agitation, etc.). Avantageusement, l'incubation est réalisée pendant une période comprise par exemple entre 15 minutes et 2 heures. Le procédé comprend en outre une étape de formation d'un complexe entre le/les composés et le/les molécules (acides nucléiques), qui peut être suivie de différentes manières, et notamment par mesure du spectre d'absorption de la solution, ainsi qu'illustré dans les exemples.

L'incubation peut être réalisée dans différents milieux, de préférence en l'absence de sérum pour éviter la dégradation des acides nucléiques avant la complexation. Il peut s'agir de solutions salines, tampons (PBS), etc., dans lesquelles les composés/acides nucléiques sont solubles. Des milieux adaptés sont par exemple les milieux DMEM, RPMI ou tout milieu compatible avec un usage in vitro, ex vivo ou in vivo. Le choix du milieu peut bien évidemment être adapté par l'homme du métier.

Les composés utilisés dans l'invention peuvent être synthétisés selon différentes voies possibles, connues de l'homme du métier. Ainsi, il est possible de synthétiser ces composés à partir de l'amphotéricine B, par couplage de la ou des parties cationiques selon les méthodes classiques de la chimie. On peut à cet égard utiliser les méthodes de synthèse décrites par Falkowski et coll. (J. Antibiot. 33 (1980) 103 ; J. Antibiot 35 (1982) 220 ; J. Antibiot. 28 (1975) 244 et J. Antibiot. 31 (1979) 1080), Schaffner et coll. (Antibiot. Chemother. 11 (1961) 724), Mechlinski et coll. (J. Antibiot. 25 (1972) 256) ou encore Pandey et coll. (J. Antibiot. 30 (1977) 158), incorporés à la présente par référence.

Il est également possible de produire ces composés en partant d'autres antibiotiques polyéniques macrolides.

Il est entendu que l'homme du métier peut adapter le procédé de préparation à l'aide de ses connaissances générales communes.

Par ailleurs, la présente invention a également pour objet de nouveaux composés dérivés d'antibiotiques macrolides polyéniques, notamment de l'amphotéricine B, doués de la capacité de vectorisation d'acides nucléiques dans les cellules ainsi que de propriétés antifongiques. Ces composés comprennent notamment une partie dérivée de l'amphotéricine B ou d'autres antibiotiques heptaéniques macrolides sur laquelle sont liés, de manière covalente, un ou plusieurs groupements ou combinaisons de groupements chargés positivement, ces groupements n'ayant pas été utilisés antérieurement pour des modifications chimiques des antibiotiques polyéniques. Ils ont été choisis notamment parmi les groupements suivants : choline, polyamine, hydrazide, N-acyl, etc. De tels composés sont représentés par exemple par les produits AMPEA, AMSA, AMDLA, AMPA, AMCE, AMPE, AMEE, SAME, PNAME, PAME, MLAME, OAMA, PAMPA et VAGA tels que définis ci-avant.

Les structures de tous les dérivés sont représentés dans les figures sous une forme ionisée, afin d'indiquer la position et le nombre de charges positives acquises à pH physiologique ou par interaction avec des acides, y compris des acides nucléiques (protonation). Les composés avec des ammoniums quaternaires portent des charges positives permanentes.

Les composés nouveaux décrits ci-dessus possèdent en outre une activité antifongique propre élevée. En effet, comme illustré dans les exemples, ces composés sont capables d'inhiber fortement la croissance (et de causer la mort) de cellules fongiques. Ces composés peuvent donc être utilisés comme agent(s) antifongique(s), en particulier pour induire la destruction de cellules fongiques in vitro, ex vivo ou in vivo. A cet égard, l'invention concerne donc également toute utilisation pharmacologique (notamment pharmaceutique) des dérivés de l'amphotéricine B décrits ci-avant. Elle concerne plus particulièrement l'utilisation de ces composés à titre d'agent(s) antifongique(s) ainsi que toute composition antifongique comprenant un tel composé. L'activité antifongique peut être utilisée sur tout type de cellule fongique exprimant de préférence un groupe ergostérol ou un précurseur correspondant, comme illustré plus loin. Les conditions de mise en oeuvre de cette activité (doses, durée, etc.) in vitro et in vivo peuvent être aisément transposées à partir de celles décrites pour l'amphotéricine B, par exemple en tenant compte des IC50.

Les composés/compositions de l'invention peuvent être utilisés pour le transfert de molécules d'intérêt dans différents types de cellules, tissus ou organes, in vitro, ex vivo ou in vivo. En particulier, ces composés/compositions peuvent être utilisés pour le transfert dans tous les types cellulaires sensibles aux antibiotiques macrolides polyéniques utilisés, notamment à l'amphotéricine B, c'est-à-dire sur lesquels ce ou ces antibiotiques exercent une activité de perméabilisation membranaire.

Préférentiellement, il s'agit de cellules contenant dans leur membrane des groupes ergosterol ou des précurseurs de celui-ci.

On peut citer par exemple les protozoaires parasites (par exemple leishmanies) ou les cellules fongiques, qui sont la cible privilégiée des antibiotiques polyéniques tels que l'amphotéricine B. Parmi les cellules fongiques on peut citer notamment les cellules de candida, de cryptococcus ou d'aspergillus.

On peut citer également des cellules de levure telles que Saccharomyces, Kluyveromyces, etc.

Par ailleurs, les composés selon l'invention sont également capables de vectoriser des molécules dans des cellules somatiques du type fibroblaste, hépatique, musculaire, nerveuse, hématopoïétique, etc. Comme le montrent les exemples, ces composés sont notamment efficaces sur des fibroblastes (3T3) et sur des cellules cancéreuses humaines (MCF-7), ce qui montre leur large spectre d'activité.

Il est entendu que dans le cadre du transfert de molécules dans les cellules, les doses de composé(s) utilisées sont de préférence non toxiques pour les cellules.

Un autre objet de l'invention concerne également les cellules modifiées par une composition telle que décrite ci-avant. Au sens de l'invention, on entend par "cellule modifiée" toute cellule comprenant une molécule d'intérêt vectorisée par une composition ou un composé tel que défini ci-avant. Comme indiqué ci-avant, les cellules peuvent être des cellules fongiques, des protozoaires parasites (par exemple : leishmanies) ou des cellules de mammifères, notamment des cellules humaines. Les cellules modifiées de l'invention peuvent être obtenues par un procédé comprenant l'incubation de cellules en présence d'une composition de l'invention, in vitro, ex vivo ou in vivo. In vitro ou ex vivo, l'incubation peut être réalisée dans tout dispositif approprié (plaque, boite, fermenteur, etc). In vivo, l'incubation peut être réalisée par administration d'une composition selon l'invention à un sujet (de préférence un mammifère), dans des conditions connues de l'homme du métier. Il peut s'agir en particulier d'une administration par voie topique, orale, parentérale, nasale, intraveineuse, intramusculaire, sous-cutanée, intraoculaire, transdermique, etc. Pour ce type d'applications, les compositions selon l'invention comprennent avantageusement un véhicule physiologiquement acceptable, tel que par exemple des solutions salines (phosphate monosodique, disodique, chlorure de sodium, potassium, calcium ou magnésium, etc, ou des mélanges de tels sels), stériles, isotoniques, ou des compositions sèches, notamment lyophilisées, qui, par addition selon le cas d'eau stérilisée ou de sérum physiologique, permettent la constitution de solutés administrables.

Les applications des compositions de l'invention sont multiples et comprennent par exemple la production de protéines recombinantes, l'étude de la régulation de l'expression de gènes, des études de marquage ou de biodisponibilité, la création d'animaux non-humains transgéniques, ou différentes applications médicales. A cet égard, les exemples montrent l'efficacité des compositions de l'invention dans le cadre de stratégies antisens dirigée contre la résistance aux antitumoraux ou dans le cadre du transfert de gènes marqueurs. Ces résultats peuvent être étendus notamment :

- à la résistance multiple aux antifongiques : les cellules fongiques étant la cible privilégiée des antibiotiques polyéniques, la vectorisation d'oligonucléotides ou de gènes dirigés contre la résistance y trouve là une application de choix. Une résistance est en effet en train d'apparaître aux nouveaux antifongiques comme les dérivés azolés et présente une menace sérieuse pour l'avenir si des mesures préventives ne sont pas menées. A cet égard, un objet particulier de l'invention réside dans l'utilisation d'une composition telle que définie ci-avant pour la préparation d'un médicament destiné au transfert, dans une cellule fongique, d'un acide nucléique réduisant les résistances aux antibiotiques de ladite cellule.
- à toute approche antisens ou anti-gène, notamment intracellulaire,
- à la production de protéines ex vivo ou in vivo, notamment de protéines choisies parmi des hormones, enzymes, facteurs de croissance, facteurs trophiques, facteurs de coagulation, lipoprotéines, lymphokines, etc;
- à la transfection pour la thérapie génique.

Les avantages des composés/compositions de l'invention sont par exemple :

- leur préparation simple et peu coûteuse,

- leur faible toxicité,
- leur activité en présence de sérum et d'antibactériens,
- une large utilisation en clinique de la Fungizone®, qui servira de référence aux études cliniques sur les dérivés,
- leur sélectivité : meilleure que la plupart de celle des effecteurs membranaires, quant à l'action sur la perméabilité des membranes : la formation de pores transmembranaires transitoires et réversibles et non pas action lytique déstabilisatrice.

La présente invention sera décrite plus en détail à l'aide des exemples qui suivent, qui doivent être considérés comme illustratifs et non limitatifs.

## DESCRIPTION DES FIGURES

**FIGURE 1 :** Structure de l'amphotéricine B.

**FIGURE 2 :** Structure des dérivés amide de l'amphotéricine B.

**FIGURE 3 :** Structure des dérivés ester de l'amphotéricine B

**FIGURE 4 :** Structure des dérivés hydrazides de l'amphotéricine B.

**FIGURE 5 :** Structure des dérivés N-alkyl de l'amphotéricine B.

**FIGURE 6 :** Structure des dérivés N-aminoacyl de l'amphotéricine B.

**FIGURE 7 :** Structure des dérivés multiples de l'amphotéricine B.

**FIGURE 8 :** Structure des dérivés d'autres polyènes macrolides cationiques.

**FIGURE 9 :** Spectre d'absorption de l'AmA ($5.10^{-6}$M) avant et après addition d'oligonucléotide (ODN, $10^{-6}$ M).

**FIGURE 10 :** Spectre d'absorption de l'AmE ($10^{-5}$M) avant et après addition d'oligonucléotide (ODN, $1,85.10^{-6}$M).

**FIGURE 11 :** Dichroïsme circulaire d'AmA ($2x10^{-5}$ M) en présence (b) et en absence (a) de pGFP.

**FIGURE 12 :** Effet de l'AmA seule ou en présence d'oligonucléotide (AS, 20 mères, 0,5μM) sur la viabilité des cellules 3T3 traitées pendant 4 h à. 37°C.

**FIGURE 13 :** Internalisation dans des cellules 3T3 d'un oligonucléotide marqué à la fluorescéine (ODN, 20-mères) selon qu'il est non-vectorisé (A), ou vectorisé par la Lipofectine® (B) ou par l'AmA (C et D).

**FIGURE 14 :** Internalisation dans des cellules MCF-7 d'un oligonucléotide marqué à la fluorescéine (ODN, 20 mères) selon qu'il est non vectorisé (A), ou vectorisé par la Lipofectine® (B), par l'AmE (C) ou par l'AmA (D).

**FIGURE 15 :** Autoradiographie du gel (polyacrylamide 20%-urée 7M) sur lequel ont été déposés de l'ODN en l'absence ou en présence (+/- = 10) d'AmA après différents temps d'incubation (0,4,8 et 16 h respectivement) dans du tampon Hépès contenant 10% (v/v) de sérum de veau foetal.

**FIGURE 16 :** Niveaux d'expression de la P-glycoprotéine (P-gp) dans les cellules 3T3 R (colonnes 1 et 3-8) ou 3T3 S (colonne 2) après quarante huit heures de traitement.

Les différents traitements, préparés dans du milieu DMEM sans sérum de veau foetal, sont les suivants :

*témoins* :

1- 3T3 R non traitées
2- 3T3 S non traitées

*recherche d'un effet antisens* :

3- 3T3 R / AS5995 (1µM) vectorisé par la Lipofectine® (20µg/mL)
4- 3T3 R / CTL (1µM) vectorisé par la Lipofectine® (20µg/mL)
6- 3T3 R / AS5995 (1µM) vectorisé par l'AmA ($5.10^{-6}$M)
7- 3T3 R / CTL (1µM) vectorisé par l'AmA ($5.10^{-6}$M)
9- 3T3 R / AS5995 (1µM) non vectorisé

*recherche d'un éventuel effet propre du vecteur:*

5- 3T3 R / Lipofectine® (20µg/mL)
8- 3T3 R / AmA $10^{-5}$M)

## EXEMPLE 1

Étude du spectre d'absorption de dérivés cationiques de l'amphotéricine B en présence d'oligonucléotides.

A un mélange de 22µl d'AmA (solution stock dans le DMSO à $7x10^{-4}$ M), et 16 µl d'un oligonucléotide 20-mères (Genosys Biotechnologies, The Woodland, England) (solution stock à $1,9 \times 10^{-4}$ M dans l'eau), ont été ajoutés 3 mL de milieu RPMI dépourvu de rouge phénol (Gibco BRL, Life Technologies, S.A., Cergy Pontoise, France). La solution a été incubée à température ambiante pendant 30 minutes. La séquence de l'oligonucléotide (CCATCCCGACCTCGCGCTCC, SEQ ID NO:1) a été choisie à partir de Alahari et al (Alahari, Dean et al. 1996) et correspond à un oligonucléotide antisens dirigé contre l'ARN du gène MDR1 et donc destiné à inhiber l'expression du gène MDR1.

Les spectres d'absorption de ces solutions ont été mesurés sur un spectrophotomètre 8452A Hewlett-Packard UV-visible.

Les spectres ont été également mesurés à partir d'un mélange de 15 µl de AmE (solution stock dans l'eau à $10^{-2}$ M), 3 mL de tampon PBS pH 7,4, et 28 µl d'un oligonucléotide 27 mères (Genosys Biotechnologies, The Woodland, England) (solution stock à $2x10^{-4}$ M). La séquence de cet oligonucléotide a été déterminée à partir de Quattrone et al. (Quattrone et al., 1994) et correspond à un oligonucléotide anti-gène dirigé contre le gène MDR1, c'est à dire un oligonucléotide capable d'inhiber l'expression du gène MDR1 (5'-TGT GTT TTT GTT TTG TTG GTT TTG TTT - 3' ; SEQ ID NO2).

Les antibiotiques polyèniques présentent des bandes d'absorption spécifiques entre 300 et 450 nm. Dans les conditions de cette expérience ($5x10^{-6}$ M d'antibiotique) l'AmE libre ou l'AmA libre présente une bande à 330 nm et un épaulement à 420 nm, caractéristiques des formes auto-associées de l'antibiotique, et quatre bandes à 345, 365, 385 et 409 nm.

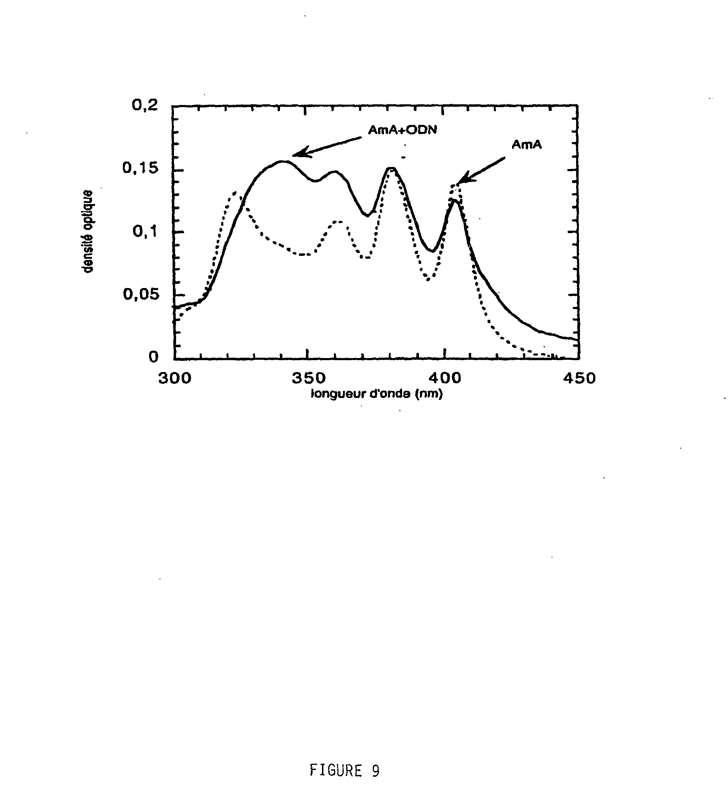

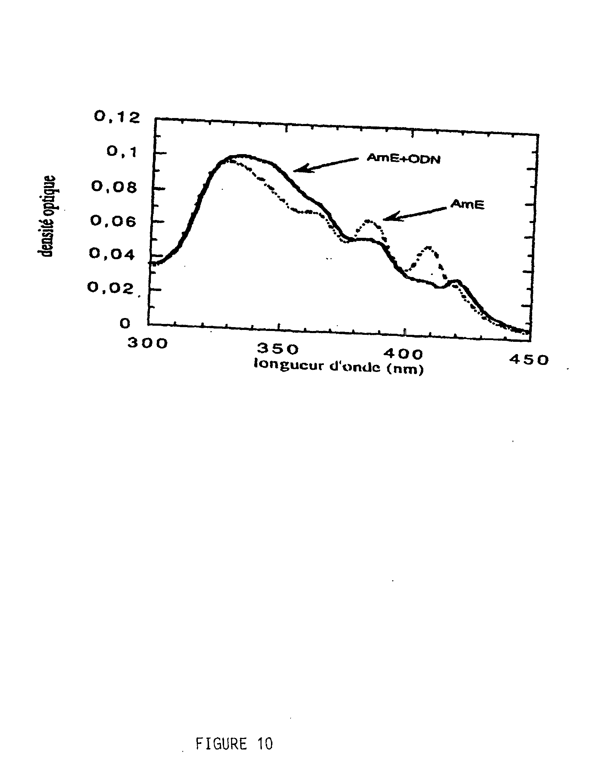

Avec l'AmA, en présence de l'oligonucléotide ($10^{-6}$ M), un bruit de fond apparaît, l'intensité des bandes de monomères à 385 et 409 nm diminue, et la bande à 330 nm est augmentée et déplacée vers le rouge (voir figure 9). Avec l'AmE, en présence de l'oligonucléotide, l'intensité des bandes à 345 et 420 nm augmente tandis que celle des bandes à 409 et 385 nm décroît (voir figure 10). Dans les deux cas, ces changements indiquent qu'une interaction entre l'antibiotique et l'oligonucléotide s'est produite et que l'état d'auto-association de l'antibiotique est modifié. Le spectre de l'oligonucléotide autour de 255 nm ne change pas, si l'augmentation de l'absorption résultant de l'augmentation du bruit de fond est prise en considération.

## EXEMPLE 2

Spectre de dichroïsme circulaire de AmA en présence du plasmide pGFP emd-c [R].

A un mélange de 30 µl d'une solution stock de AmA à $10^{-3}$ M dans le DMSO, et 6 µl de plasmide pGFP emd-c [R] (Packard, Instrument Company, Meriden, USA) (1 µg/ml), ont été ajoutés à 1,5 ml de milieu RPMI dépourvu de rouge phénol. La solution a été incubée à température ambiante pendant 30 minutes. Les spectres de dichroïsme circulaire de ces solutions ont été mesurés sur un dichrographe Jobin-Yvon Mark V.

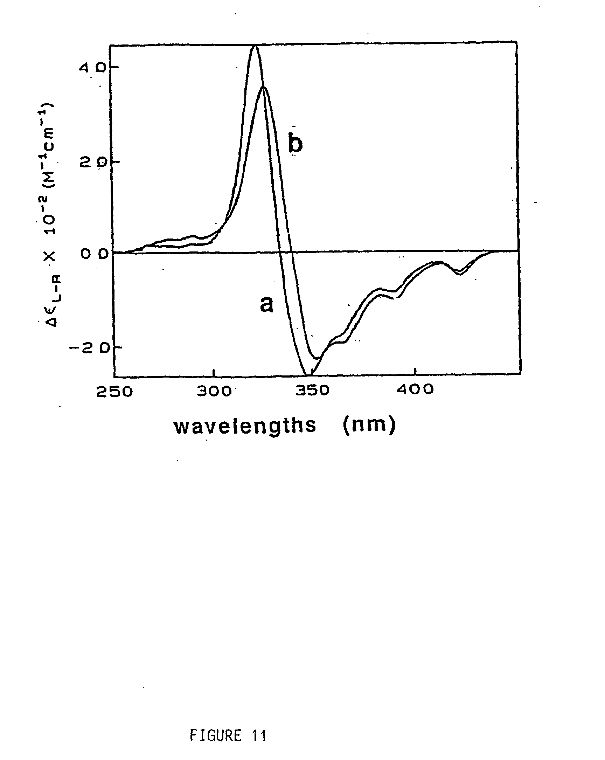

Les antibiotiques polyéniques présentent un doublet spécifique autour de 320 nm. En présence du plasmide pGFP emd-c [R], ce doublet est déplacé vers le rouge et son intensité diminue (voir figure 11). Ces caractéristiques indiquent une interaction entre AmA et le plasmide conduisant à une modification de l'état d'auto-association de l'antibiotique.

**EXEMPLE 3 :**

Étude de la toxicité de AmA en présence d'oligonucléotides antisens sur les cellules 3T3.

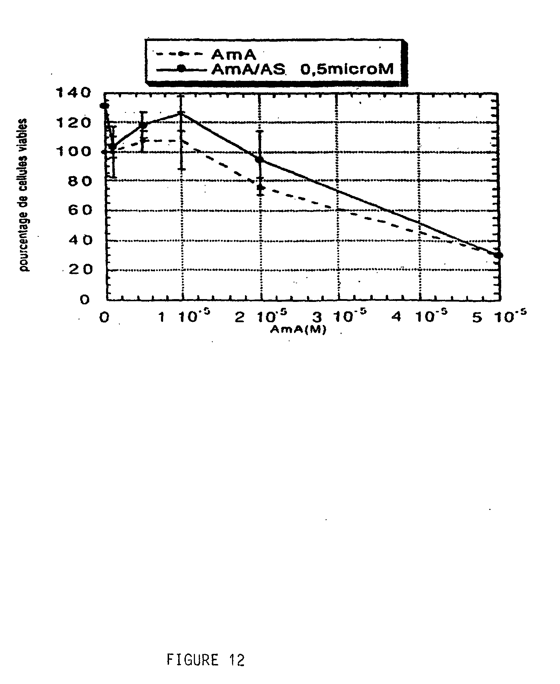

Un oligonucléotide 20-mères a été obtenu de Genosys Biotechnologies (The Woodlands, England). Sa séquence a été choisie d'après Alahari et al. et correspond à un oligonucléotide antisens permettant l'inhibition de l'expression du gène MDR1 (voir exemple 1). Les fibroblastes 3T3 ont été obtenus à l'ATCC (American Type Culture Collection).
La toxicité a été mesurée sur des solutions contenant des concentrations en AmA s'étalant de $10^{-6}$ M à $5 \times 10^{-5}$ M en présence ou en absence d'oligonucléotides à une concentration finale de $10^{-4}$ M. Dans toutes les expériences le mélange a été incubé pendant 30 minutes à température ambiante et préparé dans un milieu DMEM dépourvu de sérum. Les cellules ont ensuite été ensemencées dans un milieu DMEM et placées dans des microplaques de 96 puits à raison de $4 \times 10^4$ cellules/mL. Après 24 heures, les cellules sont rincées avec un milieu DMEM sans sérum, et 200 µl de la solution Ama/oligonucléotides préparée juste avant sont ajoutés par puits.
Après incubation pendant 4 heures à 37°, la viabilité cellulaire a été mesurée par test colorimétrique en utilisant du MTT, selon la méthodologie déjà décrite dans la littérature (Mosmann, 1983). Les résultats sont présentés sur la figure 12 et montrent que en deçà de $2 \times 10^{-5}$ M, aucune toxicité n'est observée. De plus, ces résultats montrent également que en deçà de $10^{-5}$ M, la croissance cellulaire semble être stimulée.

**EXEMPLE 4 :**

Internalisation d'oligonucléotides antisens marqués au FITC dans les cellules 3T3.

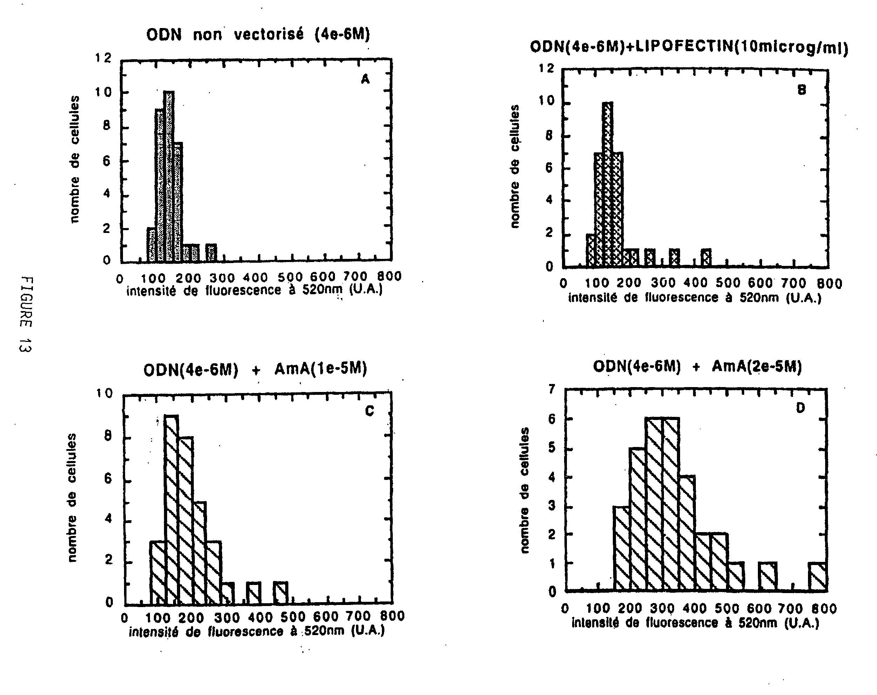

Un oligonucléotide 20-mères marqué au FITC a été obtenu de Genosys Biotechnologies (The Woodlands, England). Sa séquence a été déterminée d'après Alahari et al. (1995) et correspond à un oligonucléotide antisens permettant l'inhibition de l'expression du gène MDR1. La Lipofectine® a été obtenue de Gibco (Life Technologies, Cergy-Pontoise, France). La lipofectine® a été utilisée comme molécule de référence pour le transfert d'oligonucléotides à une concentration de 20 µg/mL dans des conditions recommandées par le fabricant. Les fibroblastes 3T3 ont été obtenus de l'ATCC.
Des aliquots d'une solution de AmA à $5,6 \times 10^{-3}$ M dans le DMSO ont été mélangés à 82 µL d'une solution d'oligonucléotides à $2,4 \times 10^{-4}$ M et à du milieu DMEM de manière à obtenir des solutions ayant une concentration en AmA de $1 \times 10^{-5}$ et $2 \times 10^{-5}$ M et un volume final de 2 mL. Les solutions ont été incubées pendant 30 minutes à température ambiante. Les cellules à 80% de confluence dans les boîtes de petri (35 mm de diamètre) ont été rincées avec du milieu sans sérum et traitées avec 2 mL de solutions AmA-oligonucléotides. Après une incubation de 4 heures, les cellules ont été rincées avec un tampon PBS et l'internalisation de l'oligonucléotide fluorescent a été détectée avec un microspectrofluorimètre à laser confocal mis au point dans le laboratoire. Les longueurs d'ondes d'excitation et d'émission étaient respectivement de 488 et 520 nm. 30 cellules ont été choisies au hasard et leur intensité de fluorescence a été mesurée. Dans la figure 13, est montrée la distribution des cellules en fonction de cette intensité (échelle de fluorescence arbitraire). Les résultats obtenus montrent qu'une internalisation beaucoup plus importante est observée en présence de AmA comparée à celle observée en présence de AmE, de lipofectine® ou d'oligonucléotides seuls.
La fluorescence apparaît comme étant distribuée de manière homogène à l'intérieur des cellules. Aucune différence significative d'intensité n'a été observée entre le noyau et le cytoplasme.

**EXEMPLE 5 :**

Internalisation d'oligonucléotides anti-gènes marqués au FITC dans les cellules MCF 7.

Un oligonucléotide de 27 mères marqué au FITC, a été obtenu de la Société Genosys Biotechnologies, The Woodlands, England). Sa séquence a été choisie d'après Quattrone et al. (1994), et correspond à un oligonucléotide anti-gène permettant l'inhibition de l'expression de MDR1 (voir exemple 1). Les cellules MCF-7 sont des cellules d'un carcinome mammaire humain.
Des aliquots d'une solution de AmE ou AmA à $10^{-3}$ M dans l'eau, ont été mélangés à 12 µL d'une solution stock d'oligonucléotides à $1,6 \times 10^{-4}$ M et à 1 mL d'un tampon Hépès de 10mM à pH 7,4, de manière à obtenir des solutions ayant une concentration en AmE ou AmA respectives de $2,7 \times 10^{-4}$ M et $1,35 \times 10^{-4}$ M. Le rapport des charges positives (du composé vecteur) sur les charges négatives (de l'acide nucléique) était de 5 dans les deux séries d'expériences. Toutes les autres conditions expérimentales étaient identiques à celles de l'exemple 4.

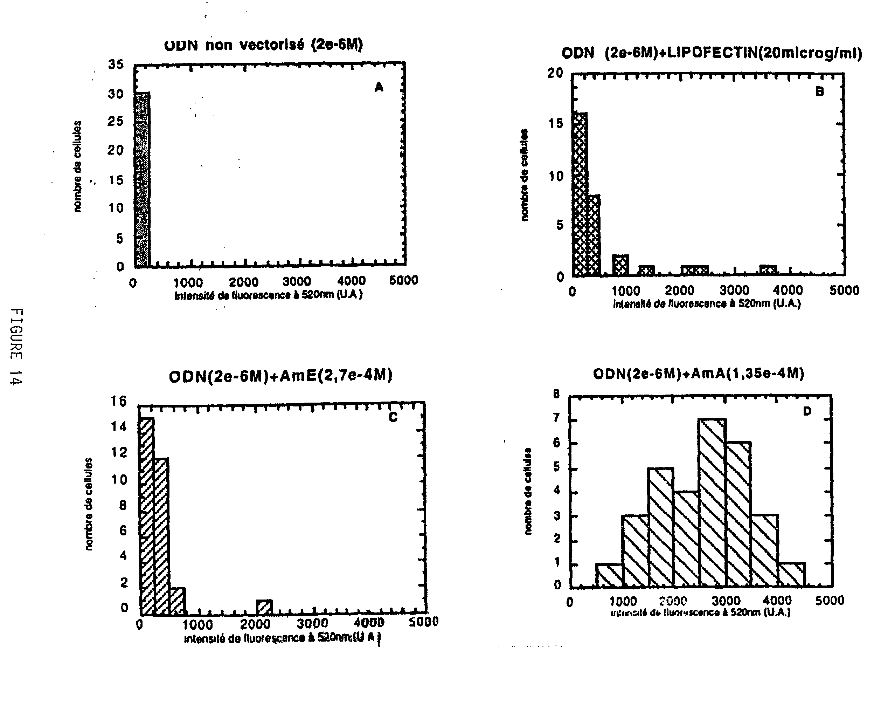

La figure 14 montre la distribution des cellules en fonction de l'intensité de fluorescence (échelle de fluorescence arbitraire). Ces résultats montrent une internalisation beaucoup plus importante en présence de AmA en comparaison à celle observée en présence de AmE, lipofectine® ou d'oligonucléotides libres.

### EXEMPLE 6

Dégradation sérique in vitro de l'oligonucléotide vectorisé.

Un oligonucléotide (ODN) de vingt sept mères est rendu radioactif par marquage en 5' au $^{32}$P selon la procédure standard (Pharmacia) utilisant la T4 polynucléotide kinase et du $^{32}(\gamma)$ATP. Une petite quantité de cet ODN marqué au $^{32}$P est mélangée à de l'ODN froid. On ajoute l'AmA à une concentration telle que l'on a un rapport des charges +/- de 10 et on laisse le complexe se former à température ambiante pendant 30 minutes. Un échantillon ne contenant pas d'AmA constitue un témoin (ODN non vectorisé). La dégradation de l'ODN est déclenchée, au temps 0, par l'addition à 37°C de 10% (V/V) de sérum de veau foetal dont l'activité enzymatique est essentiellement 3'exonucléasique (Sirotkin, Cooley et al., 1978). Des prélèvements au sein desquels on stoppe l'action des enzymes par l'addition de formamide et en mettant le tube dans la glace sont effectués au cours du temps. La réaction est ainsi arrêtée après 4, 6, 8 et 16 heures d'incubation avec le sérum. On fait ensuite migrer les échantillons sur un gel dénaturant (urée 7M) de polyacrylamide à 20%.

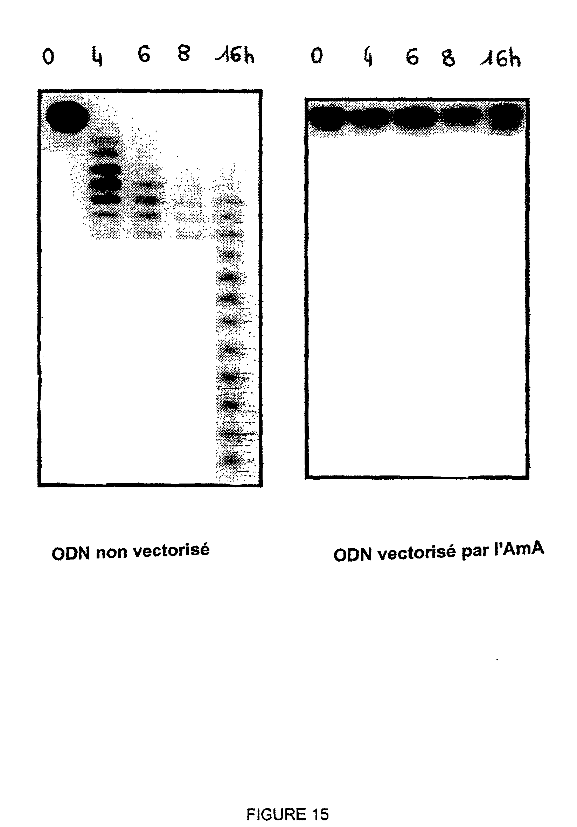

L'autoradiographie du gel révèle (Figure 15) que la dégradation sérique de l'oligonucléotide non vectorisé est très rapide, en effet au temps quatre heures la forme initiale de 27 mères a totalement disparu au profit de produits de dégradation plus courts. En revanche, la vectorisation par l'AmA permet de ralentir cette dégradation : elle ne commence qu'au temps seize heures et la forme majoritaire est encore la forme initiale de 27 mères.

Ces résultats montrent que les composés selon l'invention permettent de protéger les acides nucléiques de la dégradation par le sérum.

### EXEMPLE 7

Diminution de l'expression de la P-gp par un oligonucléotide antisens vectorisé par l'AmA dans les cellules NIH MDR-G185.

Les cellules NIH-MDR-G185 sont des fibroblastes murins 3T3 transfectés par le plasmide contenant le gène MDR1 humain (pSK1 MDR). En conséquence, ces cellules surexpriment la glycoprotéine P(P-gp) et présentent donc le phénotype de résistance multidrogue. Cet exemple montre qu'il est possible d'inhiber l'expression de cette protéine par l'utilisation de l'oligonucléotide phophorothioate antisens (AS5995) qui a pour cible l'ARN messager qui code pour la P-gp (Alahari, Dean et al. 1996).

Pour ce faire, on ensemence les cellules 3T3 R (résistantes) et 3T3 S (sensibles, non transfectées) en boîtes de pétri dans du milieu DMEM complet. Au bout de vingt-quatre heures, lorsqu'elles atteignent 80 à 90 % de confluence, elles sont traitées pendant 48 heures. Les traitements sont tous préparés dans du milieu DMEM sans sérum de veau foetal.

Au bout de 48 heures, la glycoprotéine P est quantifiée par la technique de western-blot à l'aide de l'anticorps monoclonal C219 (Dako S.A., Trappes, France). Cet anticorps, une IgG 2A kappa de souris, reconnaît un épitope intracellulaire localisé dans la partie carboxy terminale de la glycoprotéine P.

On procède tout d'abord à l'extraction des protéines. Les cellules contenues dans chaque boîte de pétri sont trypsinées, rincées par du tampon PBS et centrifugées. On ajoute à chaque culot cellulaire 100µl de tampon de lyse RIPA additionné extemporanément d'inhibiteurs de protéases. La lyse est effectuée sur la glace, pendant 30 min, en vortexant toutes les 5 minutes. Le mélange est ensuite centrifugé à 12000 tours par minute, à 4°C pendant 15 min et le surnageant contenant les protéines extraites est récupéré.

La concentration en protéines totales est déterminée par la technique de Bradford. Des échantillons contenant une quantité identique (40 mg) de protéines totales sont préparés, ils sont mis à migrer par électrophorèse en gel linéaire SDS-PAGE (Sodium Dodécyl Sulfate-polyacrylamide Gel Electrophoresis) avec une concentration finale en polyacrylamide de 7,5%.

A la fin de la migration, les protéines sont électro-transférées sur une membrane de cellulose (Immobilon-P, Millipore). La membrane est incubée pendant 1 h, dans du tampon blocking (lait écrémé 5%, TBS Tween 0,05%) afin de saturer les sites non spécifiques. On ajoute ensuite l'anticorps C219 anti-P-gp dilué au 1/200e dans du tampon blocking, pendant 2 h. Après deux lavages au TBS Tween 0,05%, une dernière incubation est réalisée avec le deuxième anticorps couplé à la peroxydase, pendant 1 h. Il s'agit d'une immoglobuline de chèvre anti-immunoglobuline de souris (Dako S.A., Trappes, France), utilisé en conditions saturantes (dilution au 1/5000e) dans du tampon blocking.

Après trois lavages au TBS-Tween 0,05 % et un lavage au Tris-HCI (pH8), la révélation est faite par chimioluminescence (kit ECL, Amersham). La quantité de P-gp est proportionnelle à l'intensité du spot.

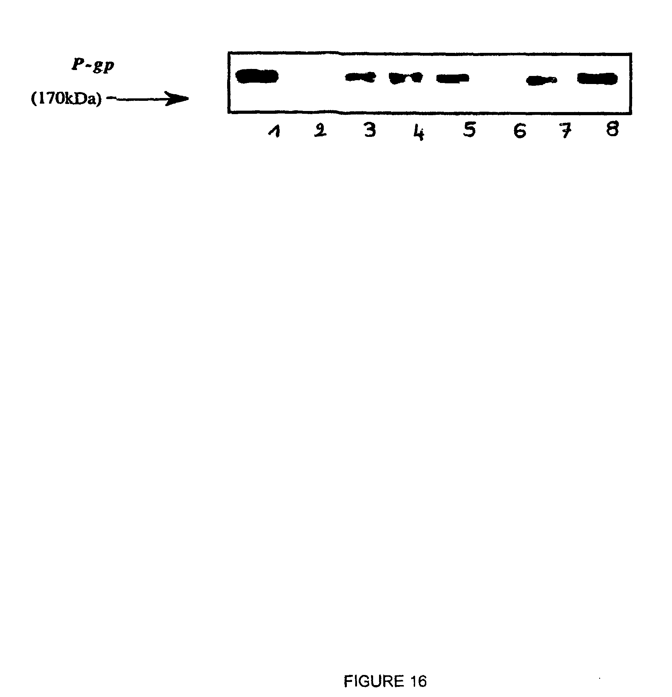

Les résultats obtenus sont présentés sur la figure 16. Ces résultats montrent que l'AS5995 inhibe l'expression de la P-gp de manière significative lorsqu'il est vectorisé par la Lipofectine®, vecteur servant de référencé, (colonne 3) ou par l'AmA à une concentration de $5.10^{-6}$M (colonne 6), alors qu'il est inactif en l'absence de vecteur (résultat non présenté ici). L'oligonucléotide contrôle (même séquence que l'AS5995 mais à l'envers) est inactif quelque soit le vecteur utilisé (colonne 4 et 7). L'apport de l'AmA par rapport à la Lipofectine® est qu'elle n'inhibe pas elle-même la synthèse de la P-gp (colonnes 5 et 8).

## EXEMPLE 8

Synthèse de dérivés amides cationiques de l'amphotéricine B.

Cet exemple décrit la synthèse de dérivés cationiques amides de l'amphotéricine B selon l'invention. Plus particulièrement, cet exemple décrit la synthèse des composés AMMEA (composé VI, Figure 2), AMPEA (composé V, Figure 2), AMPA (composé IV, Figure 2), AMSA (composé II, Figure 2) et AMDLA (composé III, Figure 2).

8.1. Synthèse de l'AMMEA

A 1 mmole (0,923 g) d'amphotéricine B dissoute dans 50 mL de DMF, 10 mmoles (1,31 mL) de 4-(2-aminoéthyl)morpholine, 10 mmoles (2,3 mL) de nitrure de diphénylphosphonyl (DPPA) et 10 mmoles (1,38 mL) de triéthylamine sont ajoutées sous agitation. Le mélange réactionnel est agité pendant une nuit à température ordinaire. Une fois la réaction achevée, on ajoute un excès d'éther diéthylique pour précipiter le produit brut. Celui-ci est centrifugé, lavé à l'éther diéthylique, séché sous vide et dissous dans du n-butanol saturé d'eau. La couche de n-butanol est lavée plusieurs fois à l'eau et réduite à un petit volume par évaporation. Le produit brut est ensuite précipité par un excès d'éther diéthylique, centrifugé, lavé plusieurs fois à l'éther et séché sous vide. L'amphotéricine B 2-(4-morpholyl)éthylamide est isolée par chromatographie sur colonne de Silicagel 60, 70-230 mesh, en utilisant le système de solvant $CHCl_3$-MeOH - $H_2O$, 10:6:1. On obtient ainsi 0,40 g (38,6%) d'amide pure ($E_{1cm}^{1\%}$ = 1150 à $\lambda$ = 382 nm dans le MeOH).

8.2. Synthèse de l'AMPEA

A une solution de 1 mmole (0,923 g) d'amphotéricine B dans 40 mL de DMF refroidie à O°C, 10 mmoles (1,2 mL) de 2-(2-aminoéthyl)pyridine, 10 mmoles (2,3 mL) de nitrure de diphénylphosphonyl (DPPA) et 10 mmoles (1,38 mL) de triéthylamine sont ajoutées sous agitation. Le mélange réactionnel est agité pendant une nuit. Le produit brut est isolé du mélange réactionnel comme décrit dans l'exemple 8.1. Pour purifier le produit brut, on le dissout dans le mélange $H_2O$ - MeOH (1:2) et on le charge sur une colonne de cellulose CM-52, on lave avec le solvant et on élue avec une solution à 5% de triéthylamine dans MeOH-$H_2O$ (2:1). Après évaporation à sec des solvants sous pression réduite, le résidu est dissous dans un petit volume de DMF et on ajoute de l'éther diéthylique pour précipiter le dérivé. Il est centrifugé, lavé à l'éther et séché sous vide. On obtient ainsi 0,37g (36%) d'amphotéricine B 2-(2-pyridyl)éthyla-mide ($E_{1cm}^{1\%}$ = 850 à $\lambda$ = 382 nm dans le MeOH).

8.3. Synthèse de l'AMPA

1 mmole (0,923 g) d'amphotéricine B dans 50 mL de DMF est mise à réagir avec 10 mmoles (1,11 mL) de 1-méthylpipérazine en utilisant les réactifs et les conditions décrits dans l'exemple 8.2. La purification du dérivé final est réalisée comme il est décrit dans l'exemple 8.2. On obtient ainsi 0,40g (40%) d'amphotéricine B 4-méthylpipérazine amide ($E_{1cm}^{1\%}$ = 1000 à $\lambda$ = 382 nm dans le MeOH).

8.4. Synthèse de l'AMSA

8.4.1. Premier protocole

A 0,5 mmole de N-Fmoc-amphotéricine B (0,573 g) dissoute dans 30 mL de DMF, 0,55 mmole de $N^1$, $N^{10}$, $N^{14}$-tris (Fmoc)-spermine (0,477g), 0,55 mmole (0,13 mL) de DPPA et 0,55 mmole (0,08 mL) de triéthylamine sont ajoutées à 0°C sous agitation. Le mélange réactionnel est laissé une nuit à température ordinaire. Une fois la réaction achevée le dérivé d'amphotéricine protégé par le F-moc est précipité par un excès d'éther diéthylique, centrifugé, lavé plusieurs fois à l'éther et séché sous vide. Le solide jaune est dissous dans un petit volume de DMF et 3 mmoles (0,4 mL) de 1,5-diazabicyclo[4.3.0]non-5-ene(DBN) dans 3mL de MeOH sont ajoutés à 0°C sous agitation pour déprotéger du Fmoc. Après 2h on ajoute de l'éther diéthylique pour précipiter le produit brut qui est ensuite centrifugé, lavé plusieurs fois à l'éther et séché sous vide. La purification est effectuée en utilisant une résine échangeuse d'ions CM-52, selon la description donnée dans l'exemple 8.2. On obtient ainsi 194 mg (30%) d'amphotéricine B N$^1$-sperminylamide ($E_{1cm}^{1\%}$ = 1000 à $\lambda$ = 382 nm dans le MeOH).

8.4.2. Deuxième protocole

A 0,5 mmole de l'ester de N-Fmoc-amphotéricine B N-hydroxysuccinimide (0,621 g) dissous dans 40 mL de DMF, 0,55 mmole (0,477 g) de N$^1$, N$^{10}$, N$^{14}$-tris (Fmoc)-spermine sont ajoutées sous agitation. Le mélange réactionnel est agité pendant une nuit à température ordinaire. La dicyclohexylurée est ensuite retirée par filtration et le filtrat est traité par un excès d'éther diéthylique pour donner un solide jaune. Les étapes suivantes ont été décrites dans l'exemple 8.4.1. On obtient ainsi 200 mg (31%) d'amphotéricine B N$^1$-sperminylamide ($E_{1cm}^{1\%}$ = 1070 à $\lambda$ = 382 nm dans le MeOH).

8.5. Synthèse de l'AMDLA

8.5.1. Premier protocole

A 0,5 mmole d'ester de N-Fmoc-amphotéricine B N-hydroxysuccinimide (0,621 g) dans 40 mL de DMF sont ajoutées sous agitation 0,55 mmole de l'ester méthylique de N$^\alpha$-Fmoc-lysyl, N$^\epsilon$-Fmoc-lysine (0,403 g). On laisse une nuit à température ordinaire. Les étapes suivantes ont été décrites dans l'exemple 8.4.2. On obtient ainsi 190 mg (32%) d'amphotéricine B (N$^\epsilon$-lysyllysine méthylester) amide ($E_{1cm}^{1\%}$ = 990 à $\lambda$ = 382 nm dans le MeOH).

8.5.2. Deuxième protocole

A 0,5 mmole de N-Fmoc-amphotéricine B (0,573 g) dans 30 mL de DMF sont ajoutées à 0°C, sous agitation, 0,55 mmole de l'ester méthylique de N$^\alpha$-Fmoc-lysyl, N$^\epsilon$-Fmoc-lysine (0,403 g), 0,55 mmole (0,13 mL) de DPPA et 0,55 mmole (0,08 mL) de triéthylamine. Le mélange réactionnel est agité pendant une nuit à température ordinaire. Les étapes suivantes sont décrites dans l'exemple 8.4.1. On obtient ainsi 150 mg (25,3%) d'amphotéricine B (N$^\epsilon$-lysyllysine méthylester) amide ($E_{1cm}^{1\%}$ = 1020 à $\lambda$ = 382 nm dans le MeOH).

## EXEMPLE 9

Synthèse de dérivés esters cationiques de l'amphotéricine B.

Cet exemple décrit la synthèse de dérivés cationiques esters de l'amphotéricine B selon l'invention. Plus particulièrement, cet exemple décrit la synthèse des composés AMPE (composé VIII, Figure 3) et AMEE (composé XXV, Figure 3).

9.1. Synthèse de AMEE

0,5 mmole (0,462 g) d'amphotéricine B sont suspendus dans 30 mL de 2-diméthylaminoéthanol sous agitation vigoureuse à 40°C et 2,5 mmoles (0,519 mg) de dicyclohexyl carbodiimide sont ajoutées. Après 5h, un petit volume de DMF est ajouté pour clarifier la solution. Le mélange réactionnel est agité pendant 24 h à 40°C. Le précipité de dicyclohexylurée est ensuite retiré par filtration, lavé avec un petit volume de DMF. Un excès d'éther diéthylique est ajouté au filtrat pour précipiter le résidu huileux. Ce résidu est centrifugé, lavé à l'éther et purifié par chromatographie sur gel de silice selon la description de l'exemple 8.1 ou par chromatographie par échange d'ion sur cellulose CM-52 selon la description de l'exemple 8.2. On obtient ainsi 100 mg (20%) d'amphotéricine B 2- diméthylaminoéthyl ester ($E_{1cm}^{1\%}$ = 1010 à $\lambda$ = 382 nm dans le MeOH).

9.2. Synthèse de AMPE

La réaction de 0,5 mmole (0,462 g) d'amphotéricine B avec du 3-diméthylaminopropanol selon le procédé décrit dans l'exemple 9.1 donne 87 mg (17,3%) d'amphotéricine B diméthylaminopropyl ester ($E_{1cm}^{1\%}$ = 1000 à $\lambda$ = 382 nm dans le MeOH).

## EXEMPLE 10

Synthèse de dérivés alkyl cationiques de l'amphotéricine B.

Cet exemple décrit la synthèse de dérivés alkyls cationiques de l'amphotéricine B selon l'invention. Plus particulièrement, cet exemple décrit la synthèse du composé SAME (composé XII, Figure 5).

0,5 mmole de N-(N'-3-diméthylaminopropyl-succinimido) amphotéricine B m(0,552g) dissous dans 40 ml de DMF sont refroidies dans un bain glacé jusqu'à 0-2°C. Une solution de diéthyléther de diazométhane est ajoutée dans un rapport 2,5 mmole $CH_2N_2$:1 mmole substrat. Le mélange réactionnel est agité pendant 1 à 2 heures à 0°C. Lorsque la réaction est terminée, l'excès de diazométhane est détruit par l'acide acétique and le diéthyléther est évaporé sous pression réduite. Le produit est ensuite précipité avec de l'éther, centrifugé, lave avec de l'éther, et seché à l'évaporeuse. On obtient ainsi 0,531g (95%) de N-(N'-3-diméthylaminopropyl-succinimido) amphotéricine B méthyl ester (SAME) ($E^{1\%}$ = 950 à 382 nm dans MeOH).

## EXEMPLE 11

Synthèse de dérivés aminoacyl cationiques de l'amphotéricine B.

Cet exemple décrit la synthèse de dérivés cationiques aminoacyls de l'amphotéricine B selon l'invention. Plus particulièrement, cet exemple décrit la synthèse des composés PAME (composé XVIII, figure 6), PNAME (composé XVII, figure 6) et MLAME (composé XVI, Figure 6).

11.1 Synthèse du N-piperidineacetyl amphotéricine B méthylester (PAME)

075 mmole (0,117g) d'acide 1-piperidinepropionique, 0,75 mmole (0,155g) de dicyclohexylcarbodiimide et 0,75 mmole (0,086g) de N-hydroxysuccinimide sont dissous dans 15 ml de DMF et agités pour la nuit à température ambiante. Lorsque la réaction est terminée, le précipité de dicyclohéxylurée est filtré, la solution est lavée avec un faible volume de DMF et le filtrat est ajouté à une solution de 20ml de DMF contenant 0,375 mmole (0,346g) d'amphotéricine B et 0,375mmole (0,055 ml) de triéthylamine. Le mélange est maintenu sous agitation pendant une nuit à température ambiante, puis un excès de diéthyéther est ajouté pour précipiter un solide jaune. Celui-ci est centrifugé, lavé plusieurs fois avec du diéthyléther et séché par évaporation. Le produit brut obtenu est dissout dans 20ml de DMF puis méthylé en utilisant une solution de diéthyléther de diazométhane, comme décrit dans l'exemple 10. La purification est réalisée par chromatographie sur colonne de gel de silice dans un système solvant $CHCl_3$-MeOH-$H_2O$ (10:6:1). On obtient ainsi 0,16g (39,7%) de PAME ($E^{1\%}$ = 1080 à 382 nm dans MeOH).

11.2. Synthèse du N-4-methyl-1-piperazinepropionyl amphotéricine B méthylester (PNAME)

0,75 mmole (0,129g) d'acide 4-methyl-1-piperazine propionique, 0,75 mmole (0,155g) de dicyclohexylcarbodiimide et 0,75 mmole (0,086g) de N-hydroxysuccinimide dans 15 ml de DMF sont agités une nuit à température ambiante. Les phases de synthèse et purification ont été réalisées comme décrit dans l'exemple 11.1. On obtient ainsi 0,143g (35%) de PNAME ($E^{1\%}$ = 1020 à 382 nm dans MeOH).

11.3. Synthèse du MLAME

A 0,5 mmole (0,462g) d'amphotéricine B et 0,5 mmole (0,07 ml) de triéthylamine dans 30 ml de DMF, on ajoute sous agitation 0,55 mmole (0,378g) de $N^{\alpha}$,$N^{\epsilon}$-di(Fmoc)-lysine N-succinimidyl ester. Le mélange réactionnel est maintenu sous agitation une nuit à température ambiante. Un excès de diéthyléther est ajouté pour précipiter le solide, qui est centrifugé, lavé plusieurs fois avec de l'éther et séché à l'évaporeuse. Le précipité est alors dissous dans un petit volume de DMF, et 2 mmole (0,25ml) de 1,5-diazabicyclo[4.3.0]non-5-ène dans le méthanol sont ajoutées à 0°C, sous agitation. Après 2 à 3 heures, du diéthyléther est ajouté pour précipiter le produit brut qui est ensuite centrifugé, lavé plusieurs fois dans l'éther et séché. Le produit est ensuite repris dans 40ml de DMF et méthylé en utilisant 5 mmole (0,47 ml) de diméthylsulfate en présence de 5 mmole (0,42g) de $NaHCO_3$, pendant 10 heures. Ensuite de l'éther diéthylique est ajouté pour précipiter le résidu huileux, qui est dissous dans 20 ml d'eau saturée avec du n-butanol, et agité en présence d'hydrocarbonate d'ammonium pendant 2 heures. Ensuite, le mélange est dilué dans l'eau et extrait au n-butanol plusieurs fois. La phase organique est lavée successivement avec de l'eau, une solution aqueuse saturée en NaCl, puis de l'eau, et concentrée dans un volume faible à basse pression. Un excès de diéthylether est ajouté pour précipiter le produit brut, qui est ensuite centrifugé, lavé plusieurs fois dans l'éther et séché à l'évaporeuse. La purification est réalisée par chromatographie d'échange d'ions sur cellulose CH-52 avec un solvant méthanol:eau (1:1) et comme effluent, une solution de méthanol à 5% NaCl:eau (1:1). L'éluat est évaporé pour éliminer le méthanol, dilué dans l'eau, et extrait plusieurs fois au n-butanol. La phase butanol est lavée plusieurs fois dans l'eau pour éliminer le NaCl, puis évaporée pour obtenir un volume faible. Le produit final est ensuite précipité avec du diéthyléther, lavé à l'éther et séché à l'évaporeuse. On obtient ainsi 0,11g (18%) de chlorure de MLAME ($E^{1\%}$ = 980 à 382 nm dans MeOH).

## EXEMPLE 12

Synthèse de dérivés combinés cationiques de l'amphotéricine B.

Cet exemple décrit la synthèse de dérivés cationiques combinés de l'amphotéricine B selon l'invention. Plus particulièrement, cet exemple décrit la synthèse des composés OAMA (composé XIX, Figure 7), OAMA L-aspartate, et PAMPA (composé XX, Figure 7).

12.1 Synthèse de la N-ornithylamphotéricine B 3-diméthylaminopropylamide (OAMA, composé XIX, figure 7)

A 0,5 mmole d'amphotéricine B 3-diméthylaminopropylamide (0,503g) dans 20 ml de DMF, on ajoute, à 0°C, 1 mmole (0,576g) de $N^{\alpha},N^{\omega}$-di-Fmoc-D-ornithine, 1,25 mmole de diphénylphosphoryl azide (0,27 ml) et 1,25 mmole de triéthylamine (0,17 ml). Le mélande réactionnel est maintenu sous agitation pendant une nuit à température ambiante. L'isolement du dérivé brut Fmoc ainsi que de son produit déprotégé et la purification du produit final sont réalisés comme décrit dans l'exemple 8.4. On obtient ainsi 0,18g (32%) de OAMA ($E^{1\%}$ = 720 à 382 nm dans MeOH).

12.2. Synthèse du sel de L-aspartate de OAMA

A 0,2 mmole (0,227g) de OAMA dans un petit volume d'eau, on ajoute sous agitation et au goutte à goutte 0,6 mmole (0,08g) d'acide L-aspartique dans 3ml d'eau. Ensuite un excès d'acétone est ajouté pour précipiter un solide jaune. Après centrifugation, lavage dans l'acétone puis dans le diéthyl éther, et séchage sous vide, 0,29g (94,4%) du sel de L-aspartate de OAMA sont obtenus ($E^{1\%}$ = 700 à 382 nm dans un mélange H2O/MeOH (1:1)).

12.3. Synthèse de N-(4-methyl-1-piperazinepropionyl) amphotéricine B 4-méthylpiperazine amide (PAMPA)

0,5 mmole (0,902g) d'amphotéricine B 4-méthylpiperazine amide dans 20 ml de DMF sont mises à réagir avec 1 mmole (0,172g) d'acide 4-méthyl-1-piperazine propionique en présence de diphénylphosphoryl azide (DPPA) et triéthylamine, dans les conditions décrites dans l'exemple 12.1. Le produit brut est isolé du mélange et purifié par chromatographie d'échange d'ions comme décrit dans l'exemple 8.2. 0,2g (35%) de PAMPA sont ainsi obtenus ($E^{1\%}$ = 850 à 382 nm dans MeOH).

## EXEMPLE 13

Mise en évidence des propriétés antifongiques des dérivés cationiques de l'amphotéricine B.

Cet exemple démontre que les dérivés cationiques de l'amphotéricine B selon l'invention présentent, en plus de leur capacité de vectorisation de molécules, des propriétés antifongiques propres.
L'activité antifongique des composés décrits dans les exemples 8 à 12 a été étudiée sur une souche de Candida albicans. Plus particulièrement, chaque composé a été incubé, à différentes concentrations, avec une culture de Candida albicans ATCC10261 (inoculum $4.10^3$ cellules/ml) en milieu liquide Sabouraud, pendant 24 heures à 30°C. La croissance cellulaire a ensuite été mesurée par spectrophotométrie à une longueur d'ondes de 660 nm. L'activité antifongique a été définie par l'IC50, c'est-à-dire la concentration de chaque produit testé induisant 50% d'inhibition de la croissance des souches. Les résultats obtenus sont présentés dans le Tableau suivant.

| Composé | IC50 ($\mu$g/ml) |
|---|---|
| AMMEA (Exemple 8) | 0,013 |
| AMPEA (Exemple 8) | 0,015 |
| AMPA (Exemple 8) | 0,015 |
| AMSA (Exemple 8) | 0,10 |
| AMDLA (Exemple 8) | 0,12 |
| AMEE (Exemple 9) | 0,08 |
| AMPE (Exemple 9) | 0,10 |
| SAME (Exemple 10) | 0,125 |
| PAME (Exemple 11) | 0,15 |
| PNAME (Exemple 11) | 0,16 |
| MLAME (Exemple 11) | 0,15 |

# EP 1 067 969 B1

(suite)

| Composé | IC50 (µg/ml) |
|---|---|
| OAMA (Exemple 12) | 0,15 |
| PAMPA (Exemple 12) | 0,17 |
| OAMA L-aspartate (Exemple 12) | 0,17 |

## BIBLIOGRAPHIE

Binet, A., Bolard, J. (1988). "Recovery of hepatocytes from attack by the pore former amphotericin B." Biochem. J. **253:** 435-440.

Brajtburg, J., W. G. Powderly, et al. (1990). "Amphotericin B: current understanding of mechanisms of action." Antimicrob. Ag. Chemother. **34:** 183-188.

Bruzzesse, T., I. Binda, et al. (1972). "Patricin methyl ester, a semisynthetic polyene antibiotic." Experientia **28:** 1515-1516.

Chéron, M., B. Cylowska, et al. (1988). "Quantitative structure-activity relationships in amphotericin B derivatives." Biochem Pharmacol. 37:827-836.

Falkowski, L., B. Stefanska, et al. (1979). "Methylesters of trimethylammonium derivatives of polyene macrolide antibiotics." J. Antibiotics **31:** 1080-1081.

Hartsel, S. And J. Bolard (1996). "Amphotericin B: New Life for an Old Drug." TIPS :

Hidaka, K., G. An, et al. (1985). "Amphotericin B enhances efficiency of DNA-mediated gene transfer in mammalian cells." Somatic Cell and Molecular Genetics **11** (2):109-115.

Jarzebski, A., L. Falkowski, et al. (1982) "Synthesis and structure-activity relationships of amides of Amphotericin B." J. Antibiotics **35**(2):220-229.

Kumar, B.V., G. Medoff, et al (1974). "Uptake of Echerichia coli DNÀ into HeLa cells enhanced by amphotericin B." Nature **250:**323-325.

Legendre, J.-Y. and F.C. Szoka Jr (1993). "Cyclic amphipatic peptide-DNA complexes mediate high efficiency transfection of adherent mammalian cells." PNAS **90:**893-897.

Legendre, J.-Y, J. Haensler, et al. (1996). "Non-viral gene delivery systems." MS Med. Sci. **12**(12): 1334-1341.

Legendre, J.Y., A. Trzeciak, et al. (1997). "Dioleoylmelittin as a novel serum-insensitive reagent for efficient transfection of mammalian cells." Bioconjugate Chemistry **8** (1): 57-63.

Malewicz, B., H.M. Jenkin, et al. (1980). "Dissociation between the induction of potassium efflux and cytostatic activity of polyene macrolides in mammalian cells." Antimicrob. Ag. Chemother. **17:**699-706.

Mechlinski, W: And S.C.P. (1972). "Polyene macrolide derivatives" I. N-acylation and esterification reactions with Amphotericin B." J. Antibiotics **25**(4):256-258.

Ohmori, N., T. Niidome, et al. (1997). "The enhancing effect of anionic alpha-helical peptide on cationic peptide-mediating transfection systems." Biochem Biophys Res Commun **235**(3):726-729.

Witzke, N. M. W. (1980). "Guanidine-type derivatives of amphotericin B." Thesis, Graduate Program in Biochemistry, Rutgers, The State University of New Jersey, New Brunswick :

Wright, J. J. K., J. A. Albarella, et al. (1982). "N-aminoacyl derivatives of polyene macrolide antibiotics and their esters." J. Antibiotics **35:**911-914.

Wyman, T. B., F. Nicol, et al. (1997). "Design, synthesis, and characterization of a cationic peptide that binds to nucleic acids and permeabilizes bilayers." Biochemistry, **36**(10):3008-3017.

Falkowski et Coll. (J. Antibiot. 33 (1980) 103 ; J. Antibiot 35 (1982) 220; J. Antibiot. 28 (1975) 244 et J. Antibiot. 31 (1979) 1080),

Schaffner et Coll. (Antibiot. Chemother. 11 (1961) 724),

Mechlinski et Coll. (J. Antibiot. 25 (1972) 256),

Pandey et al. (J. antibiot. 30 (1977) 158.

## Revendications

**1.** Composition comprenant :

- une molécule d'intérêt chargée négativement, et,
- un composé macrolide polyénique cationique susceptible d'interagir avec ladite molécule.

**2.** Composition selon la revendication 1 **caractérisée en ce que** la molécule d'intérêt chargée négativement est un acide nucléique.

**3.** Composition selon la revendication 2 **caractérisée en ce que** l'acide nucléique est un oligonucléotide antisens ou anti-gène.

**4.** Composition selon la revendication 2 **caractérisée en ce que** l'acide nucléique comprend une région codant pour un polypeptide ou une protéine d'intérêt.

**5.** Composition selon la revendication 1 **caractérisée en ce que** le composé macrolide polyénique cationique est un antibiotique macrolide heptaénique aromatique, de préférence la périmycine.

**6.** Composition selon la revendication 1 **caractérisée en ce que** le composé macrolide polyénique cationique est un dérivé d'antibiotique macrolide polyénique comprenant un ou plusieurs groupes fonctionnels cationiques ou susceptible de le devenir.

**7.** Composition selon la revendication 6 **caractérisée en ce que** l'antibiotique macrolide polyénique est un antibiotique macrolide heptanéique non aromatique, de préférence l'amphotéricine B, la candidine ou la mycoheptine.

**8.** Composition selon la revendication 6 ou 7 **caractérisée en ce que** le dérivé est un dérivé ester, amide, hydrazide, N-alkyl ou N-amino acyl.

**9.** Composition selon l'une des revendications 6 à 8 **caractérisée en ce que** le dérivé d'antibiotique macrolide polyénique comprend un ou plusieurs groupes fonctionnels cationiques liés de manière covalente sur la fonction carboxylique du groupe aglycone et/ou sur la ou les fonctions amine de l'antibiotique macrolide polyénique.

**10.** Composition selon la revendication 1 **caractérisée en ce que** le composé est un amide primaire, secondaire ou tertiaire de l'amphotéricine B.

**11.** Composition selon la revendication 1 **caractérisée en ce que** le composé est un ester de l'amphotéricine B, de préférence un choline ester ou un diméthylaminopropyl ester.

**12.** Composition selon la revendication 1 **caractérisée en ce que** le composé est un hydrazide de l'amphotéricine B, de préférence le N-méthyl pipérazine hydrazide de l'amphotéricine B.

**13.** Composition selon la revendication 1 **caractérisée en ce que** le composé est un dérivé N-alkyl de l'amphotéricine B, de préférence un dérivé N',N',N'-triméthyl ou N',N'-diméthylaminopropyl succinimidyl de l'amphotéricine B méthyl ester.

**14.** Composition selon la revendication 1 **caractérisée en ce que** le composé est un dérivé N-aminoacyl de l'amphotéricine B, de préférence le N-ornithyl-, N-diaminopropionyl-, N-lysil-, N-hexaméthyllysil-, N-pipéridine-propionyl et N,N'-méthyl-1-pipérazine-propionyl amphotéricine B méthylester.

**15.** Composition selon la revendication 1 **caractérisée en ce que** le composé est un dérivé cationique de l'amphotéricine B comportant simultanément un groupe cationique fonctionnel sur les positions carboxyl et amino de l'amphotéricine B.

**16.** Composition selon la revendication 1 **caractérisée en ce que** le composé est un dérivé cationique de la nystatine, la candidine, la mycoheptine ou la vacidine.

**17.** Composition selon l'une quelconque des revendications précédentes **caractérisée en ce que** les quantités respectives de composé et d'acide nucléique sont choisies de sorte que le rapport des charges positives du composé sur les charges négatives de l'acide nucléique (R) soit compris entre 0,1 et 20.

**18.** Dérivé cationique d'antibiotique macrolide polyénique choisi parmi l'amphotéricine B pirydilethylamide (AMPEA), l'amphotéricine B sperminylamide (AMSA), amphotéricine B N$^2$-lysyllysinemethylester amide (AMDLA), l'amphotéricine B-4-méthylpiperazine amide (AMPA), l'amphotéricine B choline ester (AMCE), l'amphotéricine B diméthylaminopropyl ester (AMPE), l'ester 2-diméthylaminoéthyl de l'amphotéricine B (AMEE), le N'N'-diméthylaminopropyl-succinimido amphotéricine B méthyl ester (SAME), le N-4-methyl-1-piperazineacétyl AME (PNAME), le N-piperidineacetyl AME (PAME), le N-tetraméthyllysil AME (MLAME), le N-ornithyl-AMA (OAMA), le N-4-méthyl-1-piperazineacetyl AMPA (PAMPA) et la Vacidine A (VAGA) telle que représentée sur la Figure 8.

**19.** Utilisation d'une composition selon l'une des revendications 1 à 17 ou d'un composé selon la revendication 18 pour le transfert de molécules d'intérêt chargées négativement dans les cellules in vitro.

**20.** Utilisation d'une composition selon l'une des revendications 1 à 17 pour la préparation d'une composition destinée au transfert d'un acide nucléique dans une cellule, un tissu ou un organe in vivo ou ex vivo.

**21.** Utilisation selon la revendication 19 ou 20 pour le transfert d'acides nucléiques dans les cellules fongiques, protozoaires parasites, et levures.

**22.** Utilisation selon la revendication 19 ou 20 pour le transfert d'acides nucléiques dans les cellules de mammifère.

**23.** Utilisation selon la revendication 19 ou 20 pour le transfert du gène MDR.

**24.** Utilisation d'une composition selon l'une des revendications 1 à 17 pour la préparation d'un produit destiné au transfert, dans une cellule fongique ou du parasite leishmania, d'un acide nucléique réduisant les résistances aux antibiotiques de ladite cellule.

**25.** Procédé de préparation d'une composition selon la revendication 1 comprenant la mise en contact, en solution, de la molécule d'intérêt avec le composé.

**26.** Procédé selon la revendication 25 comprenant en outre une étape de formation de complexes entre ladite molécule et ledit composé.

**27.** Cellule modifiée par une composition selon la revendication 1.

**28.** Utilisation d'un composé macrolide selon la revendication 1 comme agent protecteur de la dégradation des acides nucléiques par le sérum.

**29.** Sels des dérivés cationiques selon la revendication 18, de préférence des chlorures, des aspartates, des glutamates, ascorbates.

**30.** Composé selon la revendication 18, comme antifongique.


**Claims**

**1.** A composition comprising:

- a negatively charged molecule of interest; and
- a cationic polyene macrolide compound which is capable of interacting with said molecule.

**2.** A composition according to claim 1, **characterized in that** the negatively charged molecule of interest is a nucleic acid.

**3.** A composition according to claim 2, **characterized in that** the nucleic acid is an antisense or anti-gene oligonucleotide.

**4.** A composition according to claim 2, **characterized in that** the nucleic acid comprises a region coding for a polypeptide or a protein of interest.

**5.** A composition according to claim 1, **characterized in that** the cationic polyene macrolide compound is an aromatic heptaene macrolide antibiotic, preferably perimycin.

**6.** A composition according to claim 1, **characterized in that** the cationic polyene macrolide compound is a polyene macrolide antibiotic derivative comprising one or more cationic functions or is capable of becoming said derivative.

**7.** A composition according to claim 6, **characterized in that** the polyene macrolide antibiotic is a non aromatic heptaene macrolide antibiotic, preferably amphotericin B, candidin or mycoheptin.

**8.** A composition according to claim 6 or claim 7, **characterized in that** the derivative is an ester, amide, hydrazide, N-alkyl or N-amino acyl derivative.

**9.** A composition according to any one of claims 6 to 8, **characterized in that** the polyene macrolide antibiotic derivative comprises one or more cationic functional groups bonded covalently to the carboxyl function of the aglycone group and/or to the amine function or functions of the polyene macrolide antibiotic.

**10.** A composition according to claim 1, **characterized in that** the compound is a primary, secondary or tertiary amide of amphotericin B.

11. A composition according to claim 1, **characterized in that** the compound is an ester of amphotericin B, preferably a choline ester or a dimethylaminopropyl ester.

12. A composition according to claim 1, **characterized in that** the compound is a hydrazide of amphotericin B, preferably the N-methylpiperazine hydrazide of amphotericin B.

13. A composition according to claim 1, **characterized in that** the compound is an N-alkyl derivative of amphotericin B, preferably a N',N',N'-trimethyl or N',N'-dimethylaminopropyl succininimidyl derivative of amphotericin B methyl ester.

14. A composition according to claim 1, **characterized in that** the compound is is a N-aminoacyl derivative of amphotericin B, preferably N-ornithyl-, N-diaminopropionyl-, N-lysil-, N-hexamethyllysil-, N-piperdine-propionyl- or N',N'-methyl-1-piperazine-propionyl-amphotericin B methyl ester.

15. A composition according to claim 1, **characterized in that** the compound is a cationic derivative of amphotericin B simultaneously comprising a functional cationic group on the carboxyl and the amine positions of amphotericin B.

16. A composition according to claim 1, **characterized in that** the compound is a cationic derivative of nystatin, candidin, mycoheptin or vacidin.

17. A composition according to any one of the preceding claims, **characterized in that** the respective quantities of the compound and the nucleic acid are selected such that the ratio of the positive charges of the compound to the negative charges on the nucleic acid (R) is in the range 0.1 to 20.

18. A cationic polyene macrolide antibiotic derivative selected from amphotericin B pyridylethylamide (AMPEA), amphotericin B sperminylamide (AMSA), $N^2$-lysyllysinemethyl ester amide (AMDLA), amphotericin B-4-methylpiperazine amide (AMPA), amphotericin B choline ester (AMCE), amphotericin B dimethylaminopropyl ester (AMPE), the 2-dimethylaminoethyl ester of amphotericin B (AMEE), N',N'-dimethylaminopropyl-succinimido amphotericin B methyl ester (SAME), N-4-methyl-1-piperazineacetyl AME (PNAME), N-piperidineacetyl AME (PAME), N-tetramethyllysil AME (MLAME), N-ornithyl-AMA (OAMA), N-4-methyl-1-piperazineacetyl AMPA (PAMPA) and vacidin A (VAGA) as shown in Figure 8.

19. Use of a composition according to any one of claims 1 to 17 or a compound according to claim 18 to transfer negatively charged molecules of interest into cells *in vitro.*

20. Use of a composition according to any one claims 1 to 17 for the preparation of a composition for transferring a nucleic acid into a cell, a tissue or an organ *in vivo* or *ex vivo.*

21. Use according to claim 19 or claim 20, for transferring nucleic acids into fungal cells, parasitic protozoa, and yeasts.

22. Use according to claim 19 or claim 20 for transferring nucleic acids into mammalian cells.

23. Use according to claim 19 or claim 20 for transferring the MDR gene.

24. Use of a composition according to any one of claims 1 to 17 for the preparation of a product for transferring, into a fungal cell or a leishmania parasite, a nucleic acid reducing the resistance of that cell to antibiotics.

25. A method for preparing a composition according to claim 1, comprising bringing the molecule of interest into contact with the compound, in solution.

26. A method according to claim 25, further comprising a step for forming complexes between said molecule and said compound.

27. A cell modified by a composition according to claim 1.

28. Use of a macrolide compound according to claim 1 as an agent for protecting nucleic acids from degradation by serum.

**29.** Salts of cationic derivatives according to claim 18, preferably chlorides, aspartates, glutamates or ascorbates.

**30.** A composition according to claim 18, as an antifungal agent.

**Patentansprüche**

**1.** Eine Zusammensetzung, umfassend:

- ein negativ geladenes interessierendes Molekül und
- eine kationische polyene Makrolidverbindung, die fähig ist, mit dem genannten Molekül zu wechselwirken.

**2.** Zusammensetzung nach Anspruch 1, **dadurch gekennzeichnet, dass** das negativ geladene interessierende Molekül eine Nukleinsäure ist.

**3.** Zusammensetzung nach Anspruch 2, **dadurch gekennzeichnet, dass** die Nukleinsäure ein antisense- oder ein antigenes Oligonukleotid ist.

**4.** Zusammensetzung nach Anspruch 2, **dadurch gekennzeichnet, dass** die Nukleinsäure einen Bereich umfasst, der für ein interessierendes Polypeptid oder Protein codiert.

**5.** Zusammensetzung nach Anspruch 1, **dadurch gekennzeichnet, dass** die kationische polyene Makrolidverbindung ein aromatisches heptaenes Makrolidantibiotikum, vorzugsweise Perimycin ist.

**6.** Zusammensetzung nach Anspruch 1, **dadurch gekennzeichnet, dass** die kationische polyene Makrolidverbindung ein Derivat eines polyenen Makrolidantibiotikums ist, das eine oder mehrere funktionelle kationische Gruppen oder solche, die in der Lage sind, sich zu solchen zu entwickeln, umfasst.

**7.** Zusammensetzung nach Anspruch 6, **dadurch gekennzeichnet, dass** das polyene Makrolidantibiotikum ein nicht aromatisches heptaenes Makrolidantibiotikum, vorzugsweise Amphotericin B, Candidin oder Mycoheptin ist.

**8.** Zusammensetzung nach Anspruch 6 oder 7, **dadurch gekennzeichnet, dass** das Derivat ein Ester-, Amid-, Hydrazid-, N-Alkyl- oder N-Aminoacylderivat ist.

**9.** Zusammensetzung nach einem der Ansprüche 6 bis 8, **dadurch gekennzeichnet, dass** das Derivat des polyenen Makrolidantibiotikums eine oder mehrere kationische funktionelle Gruppen umfasst, die in kovalenter Weise an die Carboxylfunktion der Aglykongruppe und/oder an die Aminfunktion oder Aminfunktionen des polyenen Makrolidantibiotikums gebunden sind.

**10.** Zusammensetzung nach Anspruch 1, **dadurch gekennzeichnet, dass** die Verbindung ein primäres, sekundäres oder tertiäres Amid von Amphotericin B ist.

**11.** Zusammensetzung nach Anspruch 1, **dadurch gekennzeichnet, dass** die Verbindung ein Ester von Amphotericin B, vorzugsweise ein Cholinester oder ein Dimethylaminopropylester ist.

**12.** Zusammensetzung nach Anspruch 1, **dadurch gekennzeichnet, dass** die Verbindung ein Hydrazid von Amphotericin B, vorzugsweise das N-Methylpipe-razinhydrazid von Amphotericin B ist.

**13.** Zusammensetzung nach Anspruch 1, **dadurch gekennzeichnet, dass** die Verbindung ein N-Alkylderivat von Amphotericin B, vorzugsweise ein N',N',N'-Trimethyl- oder N',N'-Dimethylaminopropylsuccinimidylderivat des Amphotericin B-methylesters ist.

**14.** Zusammensetzung nach Anspruch 1, **dadurch gekennzeichnet, dass** die Verbindung ein N-Aminoacylderivat von Amphotericin B, vorzugsweise N-Ornithyl-, N-Diaminopropionyl-, N-Lysil-, N-Hexamethyllysil-, N-Piperidinpropionyl- und N,N'-Methyl-1-piperazinpropionyl-Amphotericin B-methylester ist.

**15.** Zusammensetzung nach Anspruch 1, **dadurch gekennzeichnet, dass** die Verbindung ein kationisches Derivat von Amphotericin B ist, welches gleichzeitig eine kationische funktionelle Gruppe an der Carboxyl- und der Aminoposition von Amphotericin B umfasst.

**16.** Zusammensetzung nach Anspruch 1, **dadurch gekennzeichnet, dass** die Verbindung ein kationisches Derivat von Nystatin, Candidin, Mycoheptin oder Vacidin ist.

**17.** Zusammensetzung nach irgendeinem der vorhergehenden Ansprüche, **dadurch gekennzeichnet, dass** die entsprechenden Mengen der Verbindung und der Nukleinsäure so ausgewählt sind, dass das Verhältnis der positiven Ladungen der Verbindung zu den negativen Ladungen der Nukleinsäure (R) zwischen 0,1 und 20 liegt.

**18.** Kationisches Derivat eines polyenen Makrolidantibiotikums, das ausgewählt ist aus Amphotericin B-pirydilethylamid (AMPEA), Amphotericin B-sperminylamid (AMSA), Amphotericin B-$N^2$-lysyllysinmethylesteramid (AMDLA), Amphotericin B-4-methylpiperazinamid (AMPA), Amphotericin B-cholinester (AMCE), Amphotericin B-dimethylaminopropylester (AMPE), dem 2-Dimethylaminoethylester von Amphotericin B (AMEE), dem N',N'-Dimethylaminopropylsuccinimido-Amphotericin B-methylester (SAME), dem N-4-Methyl-1-piperazinacetyl-AME (PNAME), dem N-Piperidinacetyl-AME (PAME), dem N-Tetramethyllysil-AME (MLAME), dem N-Ornithyl-AMA (OAMA), dem N-4-Methyl-1-piperazinacetyl-AMPA (PAMPA) und dem Vacidin A (VAGA) wie sie in Figur 8 dargestellt sind.

**19.** Verwendung einer Zusammensetzung nach einem der Ansprüche 1 bis 17 oder einer Verbindung nach Anspruch 18 zur Überführung von negativ geladenen interessierenden Molekülen in Zellen in vitro.

**20.** Verwendung einer Zusammensetzung nach einem der Ansprüche 1 bis 17 zur Herstellung einer Zusammensetzung, welche zur Überführung einer Nukleinsäure in eine Zelle, ein Gewebe oder ein Organ in vivo oder ex vivo bestimmt ist.

**21.** Verwendung nach Anspruch 19 oder 20 zur Überführung von Nukleinsäuren in Pilzzellen, Protozoenparasiten und Hefen.

**22.** Verwendung nach Anspruch 19 oder 20 zur Überführung von Nukleinsäuren in Säugetierzellen.

**23.** Verwendung nach Anspruch 19 oder 20 zur Überführung des MDR-Gens.

**24.** Verwendung einer Zusammensetzung nach einem der Ansprüche 1 bis 17 zur Herstellung eines Produkts, das zur Überführung einer Nukleinsäure, welche die Resistenzen gegenüber Antibiotika der genannten Zelle verringert, in eine Pilzzelle oder eine von einem Leishmania-Parasiten bestimmt ist.

**25.** Verfahren zur Herstellung einer Zusammensetzung nach Anspruch 1, welche das Inkontaktbringen, in Lösung, des interessierenden Moleküls mit der Verbindung umfasst.

**26.** Verfahren nach Anspruch 25, welches unter anderem einen Schritt der Komplexbildung zwischen dem genannten Molekül und der genannten Verbindung umfasst.

**27.** Zelle, welche durch eine Zusammensetzung nach Anspruch 1 modifiziert ist.

**28.** Verwendung einer Makrolidverbindung nach Anspruch 1 als Schutzmittel gegen den Abbau von Nukleinsäuren durch das Serum.

**29.** Salze der kationischen Derivate nach Anspruch 18, vorzugsweise Chloride, Aspartate, Glutamate, Ascorbate.

**30.** Verbindung nach Anspruch 18 als Fungizid.

Amphotéricine B

FIGURE 1

(i)

Amphotéricine B 3-dimethylaminopropyl amide (AMA)

(ii)

Amphotéricine B N1-sperminylamide (AMSA)

AmphotéricineB (N2-lysyllisine methylester)amide (AMDLA)

(III)

Amphotéricine B 4-methylpiperizyne amide (AMPA)

(IV)

FIGURE 2B

26

Amphotéricine B 2-(2-pyridyl)ethyl amide (AMPEA)

(V)

Amphotéricine B 2-(4-morpholyl) ethyl amide (AMMEA)

(VI)

FIGURE 2C

Amphotéricine B-choline ester (AmCE)

(VII)

Amphotéricine B 3-dimethyl-aminopropyl ester (AmPE)

(VIII)

Amphotéricine B methyl ester (AME)

(IX)

FIGURE 3A

AMEE

FIGURE 3B

(X)

Amphotéricine B 4-methyl-piperazine hydraside (HAMA)

FIGURE 4

EP 1 067 969 B1

FIGURE 5

(XI)

N,N,N-trimethyl amphotericin B methyl ester chloride (DMS-AME)

(XII)

N-(N'-3-dimethylaminopropyl-succinimido)amphotericin B methylester (SAME)

N-ornithyl amphotéricine B méthyl ester (OAME)

(XIII)

N-diaminopropionyl amphotéricine B méthyl ester (DAME)

(XIV)

FIGURE 6A

(XV) N-lysyl, amphotéricine B méthyl ester (LAME)

(XVI) N-(Nα,Nα,Nα,Nε,Nε,Nε-hexaméthyl)amphotéricine B méthyl ester chloride (MLAME)

FIGURE 6B

N-(4-méthyl-1-pipérazinepropionyl)amphotéricine B méthyl ester (PNAME)

(XVII)

N-(1-pipéridinepropionyl)amphotéricine B méthyl ester (PAME)

(XVIII)

FIGURE 6C

34

N-ornithylamphotéricine B 3-diméthylaminopropylamide (OAMA)

(XIX)

N-(4-méthyl-1-pipérazinepropionyl)amphotéricine B
4-méthylpipérazine amide (PAMPA)

(XX)

FIGURE 7

Nystatine A1 3-diméthylaminopropyl amide (NYA)

(XXI)

Candidine 3-diméthylaminopropyl amide (CAA)

(XXII)

FIGURE 8A

Vacidine A 3-diméthylaminopropyl amide (VAA)

(XXIII)

N-(N',N'-diméthylglycyl)-vacidine A 3-diméthylaminopropyl amide (VAGA)

(XXIV)

FIGURE 8B

FIGURE 9

FIGURE 10

FIGURE 11

FIGURE 12

FIGURE 13

FIGURE 14

**ODN non vectorisé**　　　　**ODN vectorisé par l'AmA**

FIGURE 15

FIGURE 16